# EUROPEAN PATENT APPLICATION

(11) **EP 4 169 940 A1**
(43) Date of publication of application: **26.04.2023**
(21) Application number: 21826416.6
(22) Date of filing: 17.06.2021
(51) Int. Cl.: C07K 16/18, C07K 16/46, C12N 15/13, C12N 15/62

(54) **IMMUNOGENICITY-REDUCED LOW-MOLECULAR ANTIBODY AND METHOD FOR PRODUCING SAME**

(30) Priority: 18.06.2020 JP 2020105104
(71) Applicant: Kyoto University, Kyoto-shi, Kyoto 606-8501 (JP)
(72) Inventor: NONAKA Motohiro, Kyoto-shi, Kyoto 606-8501 (JP); OISHI Shinya, Kyoto-shi, Kyoto 606-8501 (JP); OHNO Hiroaki, Kyoto-shi, Kyoto 606-8501 (JP); AOKI Keisuke, Kyoto-shi, Kyoto 606-8501 (JP)
(74) Representative: Hoffmann Eitle
(86) International application number: PCT/JP2021/022968
(87) International publication number: WO 2021/256524

(57) **Abstract**

The present invention relates to a low-molecular antibody composed of D-amino acids and achiral glycine, a method for producing the same, and a method for screening the low-molecular antibody using a mirror-image target protein corresponding to a target protein and an antibody library.

## Description

### Related application

This application claims priority to Japanese Patent Application No. 2020-105104 (18 June, 2020), the disclosure of which is incorporated herein by reference in its entirety.

### Technical Field

The present invention relates to an immunogenicityreduced low-molecular antibody and a method for producing the same. More specifically, the present invention relates to a low-molecular antibody composed of D-amino acids and achiral glycine, a method for producing the same, and screening of the low-molecular antibody using a mirror-image target protein corresponding to a target protein and an antibody library.

### Background Art

Monoclonal antibodies are one of the most attractive modalities in the therapeutic field. High affinity, high selectivity, and long blood half-life are the main advantages of this modality, but its size (up to 150 kDa) and structural complexity limit its scope of application. In order to solve the problems of size and stability, several antibody-like scaffolds such as Adnectin, Affibody, and Aphylline have been developed. Drug candidate molecules derived from these scaffolds have good affinity and stability despite being much smaller than conventional antibodies. However, these antibody-like scaffolds often have problems of immunogenicity, such as diminished efficacy and unanticipated side effects due to the emergence of anti-drug antibodies (ADA) .

The problems of immunogenicity exist not only with antibody-like scaffolds, but also with approved monoclonal antibody drugs. Accordingly, predicting immunogenicity and developing new drug scaffolds with reduced immunogenicity are desirable.

It is known that the protease in the body is chiral and can distinguish the structural differences between the L- and D-proteins, thus greatly improving the degradation stability in the D-proteins. Synthesis of VEGF-A inhibitors with high affinity and high stability by applying a mirror image phage display to a D-protein scaffold library has been reported. These VEGF-A inhibitors composed of D-amino acids show no immunogenicity (Non-Patent Literatures 1 and 2).

The inventors have established the screening processes for virtual mirror-image libraries and reported the development of new cancer treatments (Non-Patent Literatures 3 to 6). Further, several groups have reported mirror-image screening of phage display peptide libraries (such as Non-Patent Literatures 1 and 2 mentioned above).

VHH antibodies, which are single-domain antigen-binding fragments of camelid-specific heavy-chain-only antibodies, are one of the most attractive scaffolds for next-generation drug candidates (Non-Patent Literature 7). Despite their small size (up to 15 kDa), VHH antibodies have high specificity and high affinity similar to conventional antibodies (up to 150 kDa). Further, their high stability and high solubility are suitable for drug development. In 2019, the first VHH antibody-based drug was approved by the FDA, and several candidate molecules are undergoing clinical trials. VHH antibodies are said to have low immunogenicity, but the details are still unknown. VHH antibodies have a refolding function, and recently, production of VHH antibodies by chemical synthesis has also been reported (Non-Patent Literature 8). However, even for VHH antibodies, there is a risk that anti-drug antibodies and neutralizing antibodies may occur due to degradation of the three-dimensional structure in immune cells (Non-Patent Literatures 9 and 10).

### Citation List

### Non-Patent Literature

Non-Patent Literature 1: Mandal, K. et al., (2012) Chemical synthesis and X-ray structure of a heterochiral {D-protein antagonist plus vascular endothelial growth factor} protein complex by racemic crystallography. Proc. Natl. Acad. Sci. U. S. A. 109, 14779-14784.
Non-Patent Literature 2: Uppalapati, M. et al., (2016) A potent D-protein antagonist of VEGF-A is nonimmunogenic, metabolically stable, and longer-circulating in vivo. ACS Chem. Biol. 11, 1058-1065.
Non-Patent Literature 3: Noguchi, T. et al., (2016) Screening of a virtual mirror-image library of natural products. Chem. Commum. 52, 7653-7656.
Non-Patent Literature 4: Noguchi, T et al., (2017) Synthesis of Grb2 SH2 domain proteins for mirror-image screening systems. Bioconjugate Chem.28, 609-619.
Non-Patent Literature 5: Shu, K. et al., (2017) Synthesis of the Src SH2 domain and its application in bioassays for mirror-image screening. RSC Adv. 7, 38725-38732.
Non-Patent Literature 6: Shu, K. et al., (2019) Development of mirror-image screening systems for XIAP BIR3 domain inhibitors. Bioconjugate Chem. 30, 1395-1404.
Non-Patent Literature 7: Muyldermans, S. (2013) Nanobodies: natural single-domain antibodies. Annu. Rev. Biochem. 82, 775-797.
Non-Patent Literature 8: Hartmann, L. et al., (2019) VHH characterization. Comparison of recombinant with chemically synthesized anti-HER2 VHH. Protein Sci. 28, 1865-1879.
Non-Patent Literature 9: Papadopoulos, K.P., (2015) Unexpected Hepatotoxicity in a Phase I Study of TAS266, a Novel Tetravalent Agonistic Nanobody(R) Targeting the DR5 Receptor. Cancer Chemother Pharmacol. 75(5), 887-895.
Non-Patent Literature 10: Kibria M.G., et al. (2020) The immunogenicity of an anti-EGFR single domain antibody (V(HH)) is enhanced by misfolded amorphous aggregation but not by heat-aggregation. Eur J Pharm Biopharm. 152, 164-174.

### Summary of Invention

### Technical Problem

It is an object of the present invention to provide a new drug modality with reduced immunogenicity.

### Solution to Problem

It is known that mirror-image peptides and proteins composed of D-amino acids, which are enantiomers of natural L-amino acids, are difficult to undergo substrate recognition by in-vivo proteases. The inventors have contemplated that immunogenicity could be reduced by mirroring antibody molecules while maintaining their specificity.

In order to confirm the aforementioned concept, the inventors focused on VHH antibodies, which are known as the minimal domain having antibody functions, and established a method for chemical synthesis of mirror-image VHH antibodies composed of D-amino acids and achiral glycine using the native chemical ligation (Native Chemical Ligation: NCL) method. Then, they confirmed that the mirror-image VHH antibodies obtained had high binding specificity and reduced immunogenicity.

The present invention is based on the aforementioned findings and provides [1] to [14] below.
[1] A low-molecular antibody composed of D-amino acids and achiral glycine, the low-molecular antibody having a molecular weight of 60 KDa or less, more preferably 24 KDa or less, further preferably 15 KDa or less.

The low-molecular antibody preferably has 500 amino acids or less, more preferably 200 amino acids or less, further preferably 120 amino acids or less, specifically includes a single-domain antibody, scFv, dsFV, Fab, and Fab'.
The low-molecular antibody according to [1], wherein the low-molecular antibody is any one selected from a single-domain antibody, scFV, and Fab, preferably a single-domain antibody or scFV, more preferably a single-domain antibody.

The single-domain antibody includes a VHH antibody (including a humanized VHH antibody), an IgNAR antibody (VNAR antibody), and a camelized VH antibody.
[3] The low-molecular antibody according to [1], wherein the low-molecular antibody is a VHH antibody.
[4] The low-molecular antibody according to [1], wherein the low-molecular antibody is a VHH antibody having:
   (1) four framework regions represented by the amino acid sequences set forth in SEQ ID NOs: 1 to 4 or amino acid sequences having an identity of at least 60%, 70%, 80%, or 90% with the aforementioned sequences;
   (2) four framework regions represented by the amino acid sequences set forth in SEQ ID NOs: 5 to 8 or amino acid sequences having an identity of at least 60%, 70%, 80%, or 90% with the aforementioned sequences;
   (3) four framework regions represented by the amino acid sequences set forth in SEQ ID NOs: 9 to 12 or amino acid sequences having an identity of at least 60%, 70%, 80%, or 90% with the aforementioned sequences;
   (4) four framework regions represented by the amino acid sequences set forth in SEQ ID NOs: 13 to 16 or amino acid sequences having an identity of at least 60%, 70%, 80%, or 90% with the aforementioned sequences; or
   (5) four framework regions represented by the amino acid sequences set forth in SEQ ID NOs: 17 to 20 or amino acid sequences having an identity of at least 60%, 70%, 80%, or 90% with the aforementioned sequences.
[5] A method for producing a low-molecular antibody composed of D-amino acids and achiral glycine, the method comprising:
   1) dividing an amino acid sequence of a low-molecular antibody into a plurality of segments having a cysteine residue or an alanine residue at the N-terminus;
   2) chemically synthesizing each of the segments by the solid-phase method; and
   3) sequentially ligating the synthesized segments by the NCL method to synthesize a low-molecular antibody composed of D-amino acids and achiral glycine.
[6] The method according to [5], wherein the low-molecular antibody is any one selected from a single-domain antibody, scFV, and Fab.
[7] The method according to [5], wherein the low-molecular antibody is a VHH antibody.

In the case where the low-molecular antibody is a VHH antibody, division into four segments at two cysteines present in FR1 and FR3 and a suitable cysteine or alanine residue present therebetween is preferable. Examples of the VHH antibody that can be used include those described in [4] above.
A method for producing a low-molecular antibody composed of D-amino acids and achiral glycine against a target protein, the method comprising:
1) providing a mirror-image target protein composed of D-amino acids and achiral glycine to the target protein;
2) screening an antibody library using the mirror-image target protein, to obtain an antibody having affinity to the mirror-image target protein; and
3) synthesizing a low-molecular antibody composed of D-amino acids and achiral glycine based on an amino acid sequence of the obtained antibody.

The library is suitably a phage display library or the like. Further, affinity maturation may be performed, as required, in order to enhance the affinity of the antibody obtained in step 2).
[9] The method according to [8], wherein the synthesis of the low-molecular antibody composed of D-amino acids and achiral glycine comprises the following steps:
   1) dividing an amino acid sequence of a low-molecular antibody into a plurality of segments having a cysteine residue or an alanine residue at the N-terminus;
   2) chemically synthesizing each of the segments by the solid-phase method; and
   3) sequentially ligating the synthesized segments by the NCL method to synthesize a low-molecular antibody composed of D-amino acids and achiral glycine.
[10] The method according to [8], wherein the low-molecular antibody is any one selected from a single-domain antibody, scFV, and Fab.
[11] The method according to [8], wherein the low-molecular antibody is a VHH antibody. Examples of the VHH antibody that can be used include those described in [4] above.
[12] A polyvalent or multi-specific antibody obtained by linking low-molecular antibodies according to any one of [1] to [4] .
[13] A method for producing a polyvalent or multi-specific antibody composed of D-amino acids, comprising linking low-molecular antibodies produced by the method according to any one of [5] to [11].
[14] A kit for producing a polyvalent or multi-specific antibody composed of D-amino acids, the kit comprising one or a plurality of polypeptides each having the amino acid sequence (a) or (b) below and composed of D-amino acids and achiral glycine:
   (a) an amino acid sequence set forth in any one of SEQ ID NOs: 55 to 57, 61, and 63; or
   (b) an amino acid sequence having an identity of at least 60, 70%, 80%, 90%, 95%, 98%, or 99% with the amino acid sequence set forth in any one of SEQ ID NOs: 55 to 57, 61, and 63 (preferably in the portion corresponding to the framework regions of the sequence).

Preferably, the aforementioned kit comprises a polypeptide having an amino acid sequence having an identity of at least 60, 70%, 80%, 90%, 95%, 98%, or 99% with the amino acid sequence set forth in SEQ ID NO: 55 or SEQ ID NO: 63, or the aforementioned sequence (preferably in the portion corresponding to the framework regions of the sequence).

### Advantageous Effects of Invention

The low-molecular antibody composed of D-amino acids and achiral glycine according to the present invention has reduced immunogenicity while maintaining specificity and affinity similar to full-length antibodies. Further, it has high stability and high solubility, and is suitable for drug development, as compared with full-length antibodies.

The structure of the target protein of the low-molecular antibody of the present invention is also a complete mirror image of that of natural low-molecular antibodies composed of L-amino acids. Accordingly, screening of a low-molecular antibody composed of D-amino acids and achiral glycine specific to the target protein is enabled by screening an antibody library using the mirror-image target protein.

### Brief Description of Drawings

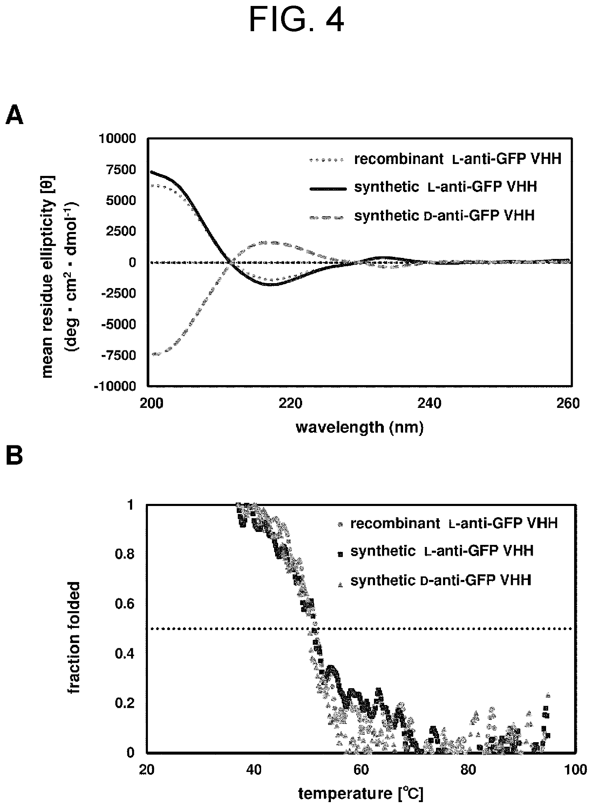

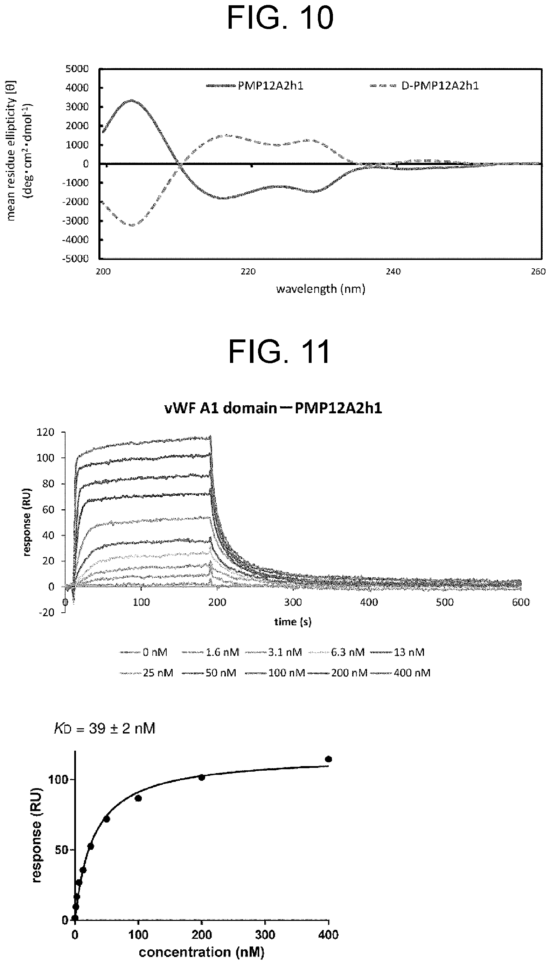

[Figure 1] Figure 1 is a conceptual diagram of the screening strategy for a virtual D-antibody library. It shows the screening of an L-antibody library using D-proteins corresponding to the screening of the D-antibody library using L-proteins.
[Figure 2] Figure 2 shows the sequences of GFP-VHH antibody, anti-HER2 VHH antibody, and caplacizumab (SEQ ID NOs: 52 to 54).
   Red: Cysteine
   Orange: Alanine
   Shaded parts: Complementarity determining regions
[Figure 3] Figure 3 shows the synthesis process of L-GFP-VHH antibody/D-GFP-VHH antibody.
   (a) Fmoc-Arg(Pbf)-OH, HBTU, HOBt, (i-Pr)₂NEt, DMF, then 20% piperidine/DMF; (b) Fmoc-Dbz-OH, HBTU, HOBt, (i-Pr)₂NEt, DMF, then 20% piperidine/DMF; (c) Fmoc-Xaa-OH, HBTU, HOBt, (i-Pr)₂NEt, DMF, then 20% piperidine/DMF [repeat (c) until the completion of the sequence]; (d) Boc₂O, (i-Pr)₂NEt, and DMF; (e) 4-nitrophenyl chloroformate and DCM; (f) (i-Pr)₂NEt and DMF; (g) TFA/H₂O/m-cresol/thioanisole/1,2-ethanedithiol (EDT) (80:5:5:5:5); (h) MESNa, 6 M guanidine and 200 mM phosphate buffer (pH 6.0); (i) Boc-Cys(Acm)-OH, HBTU, HOBt, (i-Pr)₂NEt, and DMF; (j) MPAA, 100 mM TCEP, 6 M guanidine and 200 mM phosphate buffer (pH 7.0); (k) 20 mM VA-044, 100 mM MESNa, 250 mM TCEP, 6 M guanidine and 100 mM phosphate buffer (pH 6.5); (l) PdCl₂, 6 M guanidine, 100 mM phosphate buffer, then DTT; (m) NaNO₂, 6 M guanidine, 200 mM phosphate buffer, then MPAA and TCEP [Figure 4] Figure 4 shows structural analysis of recombinant L-GFP-VHH antibody, synthetic L-GFP-VHH antibody, and synthetic D-GFP-VHH antibody by CD spectroscopy.
   (A) shows the CD spectra.
   (B) shows the evaluation of thermostability.

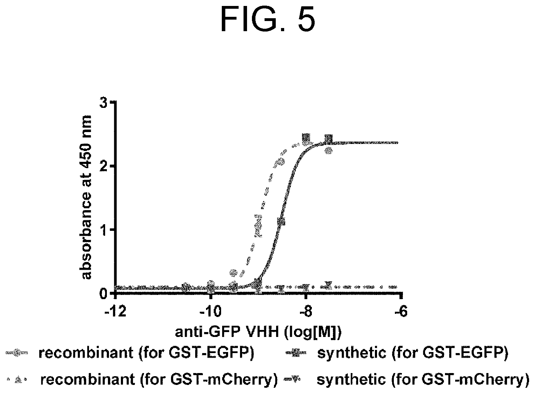

[Figure 5] Figure 5 shows the sandwich ELISA between GST-EGFP or GST-mCherry and recombinant or synthetic L-GFP-VHH antibody. It shows the average absorbance value ± SD from triplicate assays.

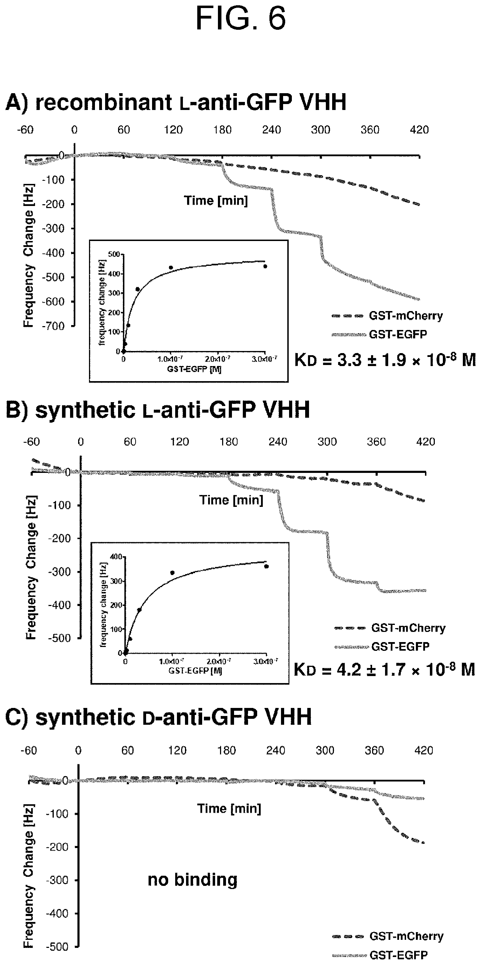

[Figure 6] Figure 6 shows the representative data of QCM analysis of GST-EGFP and GST-mCherry against recombinant L-GFP-VHH antibody, synthetic L-GFP-VHH antibody, and synthetic D-GFP-VHH antibody. In order to determine the binding affinity from triplicate assays, Langmuir model was used.

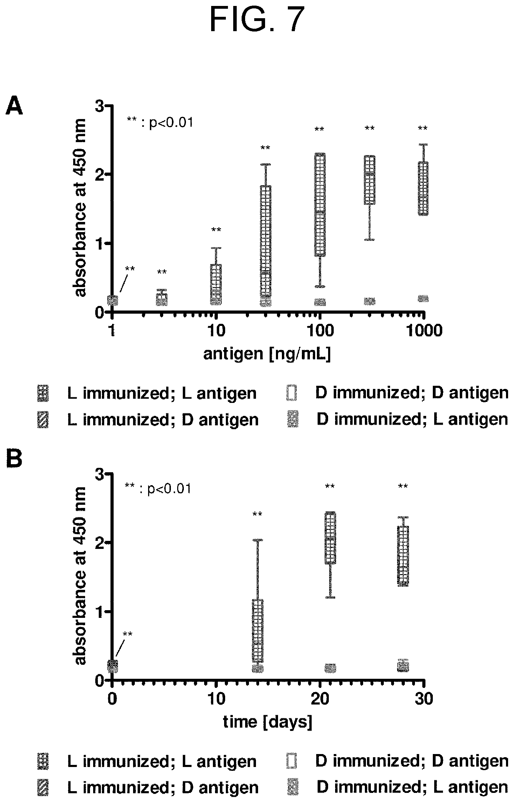

[Figure 7] Figure 7 shows the immunogenicities of synthetic L-GFP-VHH antibody and synthetic D-GFP-VHH antibody.
   (A) ELISA for ADA measurement at various antigen concentrations. The serum collected on day 28 was assayed.
   (B) ELISA for ADA measurement of serum collected on day 0, 14, 21, and 28. The concentration of coated antigen is 1000 ng/mL. It shows the statistically significant difference calculated by the Mann-Whitney U test for ADA production in synthetic L-GFP-VHH antibody and synthetic D-GFP-VHH antibody-administered mice.

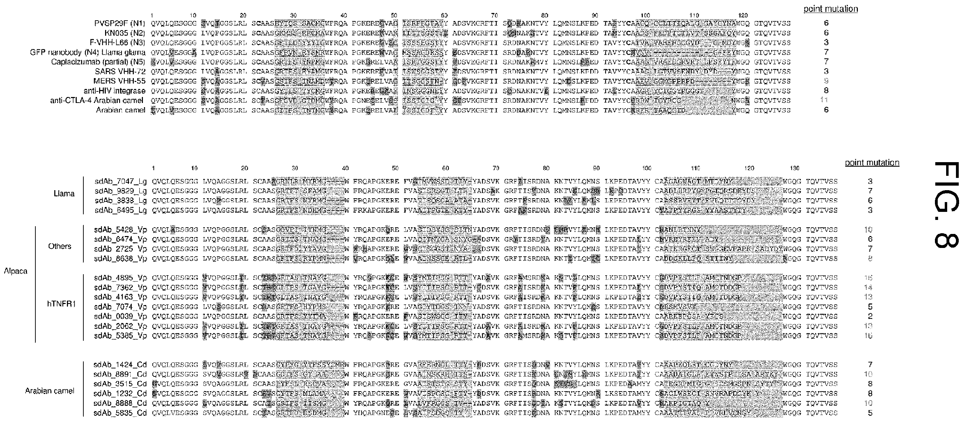

[Figure 8] Figure 8 shows the sequence alignment of a known VHH antibody. The three CDR regions are shaded, and the mutation sites in the FR regions are highlighted. As obvious from the figure, the FR regions include 16 mutations at maximum. Each sequence is set forth in SEQ ID NOs: 21 to 51 in the Sequence Listing sequentially from top to bottom.

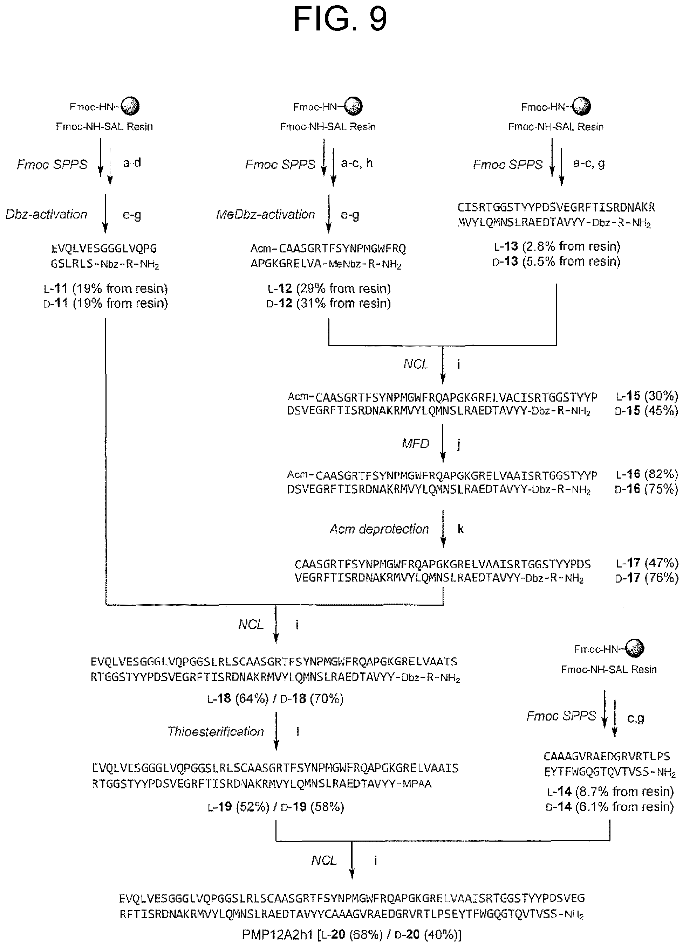

[Figure 9] Figure 9 shows the synthesis process of PMP12A2h1/D-PMP12A2h1.
   (a) Fmoc-Arg(Pbf)-OH, HBTU, HOBt, (i-Pr)₂NEt, DMF, then 20% piperidine/DMF; (b) Fmoc-Dbz-OH or Fmoc-MeDbz-OH or Fmoc-(o-Boc)Dbz-OH, HBTU, HOBt, (i-Pr)₂NEt, DMF, then 20% piperidine/DMF; (c) Fmoc-Xaa-OH, Oxyma Pure, DIC, DMF, then 20% piperidine/DMF [repeat (c) until the completion of the sequence]; (d) Boc₂O, (i-Pr)₂NEt, and DMF; (e) 4-nitrophenyl chloroformate and DCM; (f) (i-Pr)₂NEt and DMF; (g) TFA/H₂O/m-cresol/thioanisole/1,2-ethanedithiol (EDT) (80:5:5:5:5); (h) Boc-Cys(Acm)-OH, Oxyma Pure, DIC, and DMF; (i) MPAA, TCEP, 6 M guanidine and 200 mM phosphate buffer (pH 7.0); (j) VA-044, MESNa, TCEP, 6 M guanidine and 200 mM phosphate buffer (pH 6.5); (k) PdCl₂, 6 M guanidine, 200 mM phosphate buffer, then DTT; (l) NaNO₂, 6 M guanidine, 200 mM phosphate buffer, then MPAA and TCEP
[Figure 10] Figure 10 shows the CD spectra of PMP12A2h1 (solid line) and D-PMP12A2h1 (dashed line).
[Figure 11] Figure 11 shows the analysis results of the binding affinity between pmp12a2h1 and vWF A1 domains by surface plasmon resonance. The graph shows data when PMP12A2h1 was run at 400 nM, 200 nM, 100 nM, 50 nM, 25 nM, 13 nM, 6.3 nM, 3.1 nM, 1.6 nM, and 0 nM. Dissociation constant K_{D} = 39 ± 2 nM

### Description of Embodiments

### 1. Low-molecular antibody of the present invention "Low-molecular antibody"

In this description, the "low-molecular antibody" means an antibody, an antibody fragment, or an antibody-like molecule having a low molecular weight than a full-length antibody (in the case of IgG, about a molecular weight of 150 KDa) but having affinity and specificity to an antigen.

The target antigen (target protein) of the low-molecular antibody of the present invention is not specifically limited but is preferably a low-molecular antibody that recognizes a tumor-associated antigen, an inflammation- or allergy-associated antigen, a virus or bacteria infection-associated antigen, or a cardiovascular disease-associated antigen, in consideration of therapeutic and diagnostic applications.

The molecular weight of the low-molecular antibody of the present invention is preferably 60 KDa or less, more preferably 24 KDa or less, further preferably 15 KDa or less.

The amino length of the low-molecular antibody of the present invention is preferably 500 amino acids or less, more preferably 200 amino acids or less, further preferably 120 amino acids or less.

Examples of the low-molecular antibody according to the present invention can include a single-domain antibody, scFv, dsFV, Fab, and Fab'.

Preferably, the low-molecular antibody is a single-domain antibody, scFV, and Fab, more preferably a single-domain antibody or scFV, further preferably a single-domain antibody. As will be described later, the present invention includes polyvalent antibodies or multi-specific antibodies obtained by linking and multimerizing these low-molecular antibodies such as diabody (dimer of scFV) and F(ab')² (dimer of Fab'). In these multimers, the average molecular weight and the amino acid length of the monomers constituting them are preferably in the aforementioned ranges.

### "Single-domain antibody"

Single-domain antibody (SDAB: Single Domain Antibody) is an antibody molecule composed of single domain (single variable domain) having antigen-binding properties. Examples thereof can include VHH antibodies designed from the heavy-chain antibodies of camelids (such as camels, llamas, and alpacas) (including humanized VHH antibodies), IgNAR antibodies designed from the heavy-chain antibodies of cartilaginous fish (such as sharks) (VNAR antibodies), and camelized VH antibodies.

### "VHH antibody"

The single-domain antibody is preferably a VHH antibody. A VHH (Variable domain of Heavy chain of Heavy-chain antibodies) antibody is a low-molecular antibody derived from a variable region of a heavy-chain antibody composed only of camelid H chains and is also called Nanobody (R).

Since the VHH antibody has an average molecular weight of about 15 kDa and an amino acid length of about 110 to 120 amino acids, it can be produced recombinantly in a conventional microbial expression system, and as mentioned above, production by chemical synthesis has also been reported.

The VHH antibody has the property of being highly thermostable and highly reversible in its three-dimensional structure, and easily refolding to return to the original three-dimensional structure even when denatured by heat. In addition, it is said to be generally less immunogenic and safer than full-length antibodies.

A VHH antibody is composed of three CDR regions (CDR1, CDR2, and CDR3) that are important for antigen binding and four conserved framework regions (FR1, FR2, FR3, and FR4) surrounding these CDR regions. The configuration of a VHH antibody:

FR1-CDR1-FR2-CDR2-FR3-CDR3-FR4

Muyldermans et al. reported that camel-derived VHH antibodies have FR1 at positions 1 to 30, CDR1 at positions 31 to 35, FR2 at positions 36 to 49, CDR2 at positions 50 to 65, FR3 at positions 66 to 94, CDR3 at positions 95 to 102, FR4 at positions 103 to 113, according to the EU numbering of Kabat et al. (L Riechmann and S Muyldermans, J Immunol Methods. 1999 Dec 10; 231 (1-2): 25-38). However, these positions follow the EU numbering of Kabat et al., may differ in VHH antibodies from other animals, and may not match the positions in the actual sequences. Further, they may differ in the IMGT numbering or other numbering.

In an embodiment, FR1, FR2, FR3, and FR4 each have the following sequence (framework regions of the VHH antibody used in Examples).
FR1: QVQLVESGGALVQPGGSLRLSCAAS (SEQ ID NO: 1)
FR2: WYRQAPGKEREWVA (SEQ ID NO: 2)
FR3: YEDSVKGRFTISRDDARNTVYLQMNSLKPEDTAVYYCN (SEQ ID NO: 3)
FR4: WGQGTQVTVSS (SEQ ID NO: 4)

In another embodiment, FR1, FR2, FR3, and FR4 each have the following sequence (framework regions of llama VHH antibody (sdAb_7047_Lg)).
FR1: QVQLQESGGGLVQAGGSLRLSCAAA (SEQ ID NO: 5)
FR2: WFRQAPGKEREFVG (SEQ ID NO: 6)
FR3: YADSVKGRFIISRDNAKNTVYLQMNSLKPEDTAVYYCA (SEQ ID NO: 7)
FR4: WGQGTQVTVSS (SEQ ID NO: 8)

In another embodiment, FR1, FR2, FR3, and FR4 each have the following sequence (framework regions of alpaca VHH antibody (sdAb_6474_Vp)).
FR1: QVQLQESGGGLVQPGGSLRLSCAAS (SEQ ID NO: 9)
FR2: WYRQAPGKERELVA (SEQ ID NO: 10)
FR3: DSVKGRYTISRDYAKNTVYLQMNSLKPEDTALYYCN (SEQ ID NO: 11)
FR4: WGQGTQVTVSS (SEQ ID NO: 12)

In another embodiment, FR1, FR2, FR3, and FR4 each have the following sequence (framework regions of alpaca VHH antibody (sdAb_0039_Vp)).
FR1: QVQLQESGGGLVQPGGSLRLSCAAS (SEQ ID NO: 13)
FR2: WFRQAPGKEREFVA (SEQ ID NO: 14)
FR3: YADSVKGRFTISRDNAKNTVYLQMNSLKPEDTAVYYCA (SEQ ID NO: 15)
FR4: WGQGTQVTVSS (SEQ ID NO: 16)

In another embodiment, FR1, FR2, FR3, and FR4 each have the following sequence (framework regions of camel VHH antibody (sdAb_5835_Cd)).
FR1: QVQLVESGGGSVQAGGSLRLSCTAS (SEQ ID NO: 17)
FR2: WYHQAPGNECELVS (SEQ ID NO: 18)
FR3: YADSVKGRFTISRDNAKNTVYLQMNSLKPEDTAMYYCA (SEQ ID NO: 19)
FR4: WGQGTQVTVSS (SEQ ID NO: 20)

The aforementioned sequences are just examples, and those skilled in the art would be able to appropriately use the sequences of the framework regions of known VHH antibodies described in Figure 8 and other known VHH antibodies registered in a database (Single Domain Antibody Database: http: //www.sdab-db.ca/?).

Unlike CDR regions, framework regions can be altered, such as conservative substitutions, without adversely affecting the binding properties of antibodies (Mitchell LS. and Colwell LJ., Proteins (2018) 86 (7): 697-706). Further, techniques for improving the stability and specificity of antibodies by modifying framework residues important for stabilizing antibody molecules and antigen-binding sites are also known in the art.

It is also well-known in the art that a polypeptide having an amino acid sequence modified by deletion, addition, and/or substitution of one or several amino acid residues with respect to a certain amino acid sequence maintains its biological activity (Mark, D. F. et al., Proc. Natl. Acad. Sci. USA (1984) 81, 5662-5666, Zoller, M. J. and Smith, M., Nucleic Acids Research (1982) 10, 6487-6500, Wang, A. et al., Science 224, 1431-1433, Dalbadie-McFarland, G. et al., Proc. Natl. Acad. Sci. USA (1982) 79, 6409-6413).

For example, when classified based on the properties of side chains, amino acids classified into the same group in the amino acid sequence constituting a certain polypeptide are highly likely to maintain the activity of the polypeptide even if they are substituted with each other. Substitutions between amino acids within such groups are referred to as conservative substitutions.
Hydrophobic amino acids (A, I, L, M, F, P, W, Y, and V),
Hydrophilic amino acids (R, D, N, C, E, Q, G, H, K, S, and T),
Amino acids having aliphatic side chains (G, A, V, L, I, and P),
Amino acids having hydroxyl group-containing side chains (S, T, and Y),
Amino acids having sulfur atom-containing side chains (C and M),
Amino acids having carboxylic acid and amide-containing side chains (D, N, E, and Q),
Amino acids having base-containing side chains (R, K, and H),
Amino acids having aromatic-containing side chains (H, F, Y, and W)

Accordingly, the framework regions may be combinations of amino acid sequences at least having an identity of 60%, 70%, 80%, 90%, or 95% with FR1, FR2, FR3, and FR4 set forth in SEQ ID NOs: 1 to 4, SEQ ID NOs: 5 to 8, SEQ ID NOs: 9 to 12, SEQ ID NOs: 13 to 16, or SEQ ID NOs: 17 to 20 above, respectively, as long as they have a predetermined antigen specificity.

Further, the framework regions may be combinations of amino acid sequences having one to several, preferably 1 to 20, 1 to 10, 1 to 5, 1 to 4, 1 to 3, 1 or 2 deletions, substitutions, additions, or insertions relative to FR1, FR2, FR3, and FR4 set forth in SEQ ID NOs: 1 to 4, SEQ ID NOs: 5 to 8, SEQ ID NOs: 9 to 12, SEQ ID NOs: 13 to 16, or SEQ ID NOs: 17 to 20 above, respectively, as long as they have a predetermined antigen specificity.

CDR regions are determined depending on the target protein. A new VHH antibody can be designed by grafting the CDR region of a known VHH antibody against a target protein or a VHH antibody against a target antigen found by screening described below to the above FR regions.

In the present invention, the "target protein" is a protein to which a low-molecular antibody (such as a VHH antibody) specifically binds, regardless of its size. In this description, the expression "target protein" may be used interchangeably with the terms target peptide, target polypeptide, and target antigen.

### "D-amino acids"

The low-molecular antibody of the present invention is composed of D-amino acids. However, glycine has no asymmetric carbon and does not have D-type and L-type stereoisomerism (achiral glycine). Therefore, more precisely, the low-molecular antibody of the present invention is composed of D-amino acids and achiral glycine. In this description, this may be simply described as "being composed of D-amino acids", but such description includes "being composed of D-amino acids and achiral glycine".

Amino acids constituting living organisms are L-type, and D-amino acids are extremely limited, such as the peptidoglycan of bacteria. A protein composed of D-amino acids (D-type or D-protein) has a mirror image structure with respect to a protein having the same sequence composed of L-amino acids (L-type or L-protein) (Mandal, K. et al. and Uppalapati, M. et al., mentioned above). It has been reported that the D-protein is resistant to enzymatic degradation and is non-immunogenic because the enzymes present in living organisms distinguish between the L-type and D-type and act on the L-protein in a stereospecific manner.

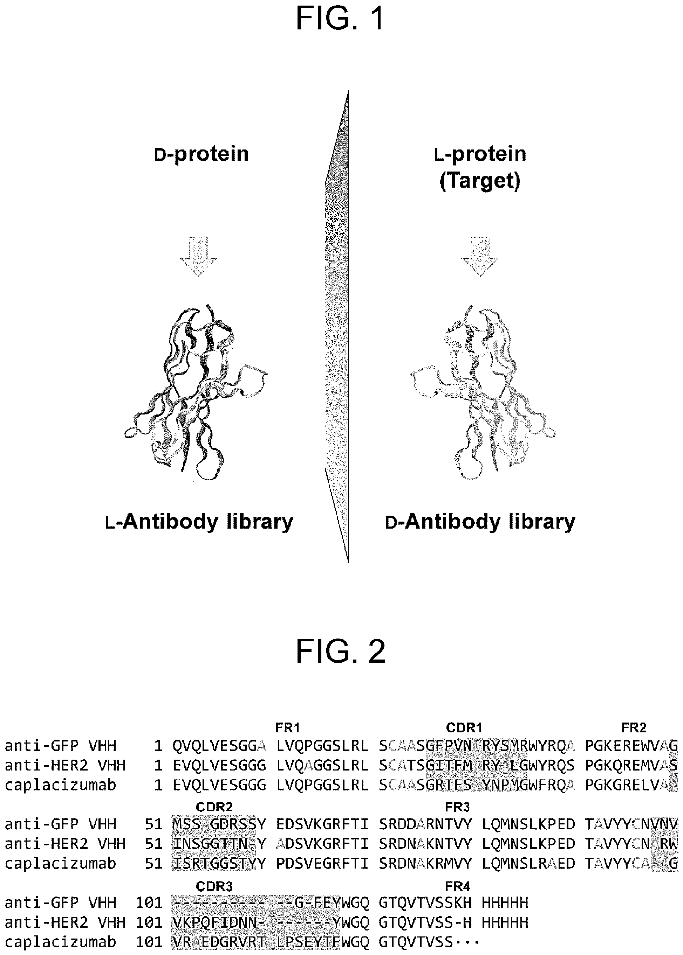

The inventors have confirmed that a low-molecular antibody composed of D-amino acids is in a mirror-image relationship to a low-molecular antibody composed of L-amino acids. As a result of various studies to apply D-VHH antibodies (Figure 1), which are capable of acting on a target protein present in living organisms, to drug discovery research, they have established a chemical synthesis process for D-VHH antibodies and confirmed that D-VHH antibodies are non-immunogenic.

### 2. Synthesis of low-molecular antibody of the present invention

The low-molecular antibody of the present invention can be chemically synthesized using D-amino acids and achiral glycine. Since there is a limit to the length of amino acids that can be synthesized by the solid-phase method (SPPS), the amino acid sequence of the low-molecular antibody is divided into several sequences of several segments and synthesized segments are sequentially ligated to synthesize the low-molecular antibody. Although each segment can be ligated by a known peptide ligation method, it is preferable to use the native chemical ligation (NCL) method.

### "NCL (native chemical ligation) method"

The NCL method is a technique for ligating two unprotected peptide segments by only mixing a peptide having an α-thioester at the C-terminus and a peptide having an unprotected cysteine at the N-terminus under mild conditions (pH7 and 20°C to 37°C) to promote the reaction (Dawson, P.E.; Muir, T.W.; Clark-Lewis, I.; Kent, S.B.H. Science, 1994, 266, 776.). Since this method utilizes functional groups inherent in peptides, it does not require a special activating agent, and the reaction proceeds in good yield even with peptides having unprotected side chains. In the NCL method, ligation sites containing cysteine residues in the target amino acid sequence must be present at appropriate intervals. However, methods have also been developed to substitute alanine residues for cysteine residues in the case where proteins have very few cysteine residues or where cysteine residues are not present at appropriate positions (Johnson, E. C. B.; Kent, S. B. H. J. Am. Chem. Soc. 2006, 128, 6640). According to the NCL method, peptide chains of up to about 200 residues can be chemically synthesized.

The method for producing the low-molecular antibody of the present invention comprises the following steps:
1) dividing an amino acid sequence of a low-molecular antibody into multiple peptide segments having a cysteine residue or an alanine residue at the N-terminus;
2) chemically synthesizing each of the segments by the solid-phase method; and
3) sequentially ligating the synthesized segments by the NCL method to synthesize a low-molecular antibody composed of D-amino acids and achiral glycine.

Each segment generally has 50 amino acids or less, preferably 40 amino acids or less, more preferably 30 amino acids or less, due to the limitation of chemical synthesis by the solid-phase method. The solid-phase method to be used is not specifically limited, and known methods such as the Boc method and the Fmoc method can be used, where the Fmoc method is preferable. For ligation by the NCL method, the N-terminus of each segment is desirably a cysteine residue, but when a cysteine residue is not present at a suitable position, an alanine residue is used, as mentioned above.

In the case where each segment synthesized has low solubility, it is desirable to attach a solubility tag at a terminus. As a solubility tag, a His tag is preferable, for example.

In the case of a VHH antibody, two cysteine residues are present in framework regions FR1 and FR3 (Figure 2). However, three segments divided by these two cysteine residues have a length of the central segment exceeding 70 amino acids and are difficult to be chemically synthesized at once. Therefore, in addition to the aforementioned two cysteine residues, synthesis is performed by dividing the whole into four segments having 50 amino acids or less using an alanine residue present at a suitable position between them or an alanine or cysteine residue in the CDR regions and ligating the segments by the NCL method. In Examples described below, the whole was divided into four segments at Cys²², Cys⁹⁶, and Ala⁵⁴ (the numerical values are positions in the Sequence Listing). These are just examples, and the second division point is appropriately set according to the structure (sequence) of the VHH antibody, in particular.

The ligation order of each segment is appropriately determined according to the structure of the antibody to be synthesized and each segment. In Examples described below, although the segments divided into two at the Ala residue are ligated first, the segments at both ends may be ligated and finally the Ala portion may be ligated. In the segment condensation reaction, isolation of the resulting thioester intermediate is preferred.

The synthesized low-molecular antibody is denatured using urea or the like, and then dialyzed or diluted to spontaneously refold to form a correct three-dimensional structure.

### 3. Screening of low-molecular antibody of the present invention

Based on the mirror-image relationship of the substrate specificity of an L-protein and a D-protein, drug discovery screening using a virtual mirror-image library is possible. Specifically, a drug discovery candidate can be selected by (1) first chemically synthesizing a D-type target protein molecule (D-protein),
(2) screening a chiral natural product using the synthetic D-protein,
(3) synthesizing a mirror image of the hit compound (L-protein), and
(4) evaluating the biological activity of the L-protein.

### "Screening using mirror-image target protein"

A low-molecular antibody composed of D-amino acids is in a complete-mirror-image relationship to a low-molecular antibody composed of L-amino acids and, and its target antigen is also in a complete-mirror-image relationship. Accordingly, application of screening using the aforementioned virtual mirror-image library enables screening of a low-molecular antibody composed of D-amino acids against a natural target protein.

The present invention also provides a method for producing a low-molecular antibody composed of D-amino acids against a target antigen protein using the aforementioned strategy. Specifically, the method comprises the following steps of:
1) providing a mirror-image target protein composed of D-amino acids and achiral glycine against a target protein;
2) screening an antibody library using the mirror-image target protein, to obtain an antibody having affinity to the mirror-image target protein; and
3) synthesizing a low-molecular antibody composed of D-amino acids and achiral glycine based on the amino acid sequence of the antibody obtained.

The antibody library to be used can be an antibody library known in the art such as a phage display library and may be any one of an immune library, a naive library, and a synthetic library. Phage display libraries have already been constructed also for low-molecular antibodies such as scFv and VHH antibodies, and these can be preferably used or prepared according to conventional methods.

In the case of using a phage display library, phage selection (biopanning) can be performed with the D-target protein in the same manner as with conventional target antigens. That is, the library is reacted with the immobilized target protein, and after washing and removing unbound phages, the bound phages are eluted and grown in E. coli repeatedly to concentrate phages specific to the target protein. For immobilization, immobilization by adsorption or immobilization using biotin-streptavidin can be used.

The selected low-molecular antibody may optionally undergo affinity maturation to increase affinity, as required. Affinity maturation can be either in vivo or in vitro, preferably in vitro. In the case of in vitro, mutations are introduced into antibody genes by known methods such as chain shuffling, random mutagenesis by error-prone PCR, and CDR walking to create a secondary library, from which antibodies with high affinity may be screened.

The sequence of the selected natural-type low-molecular antibody is determined, and a low-molecular antibody composed of D-amino acids is chemically synthesized based on the sequence. Chemical synthesis can be performed by the NCL method according to the description in the preceding section "3. Synthesis of low-molecular antibody of the present invention".

As mentioned above, the CDR regions are determined according to the target protein. Accordingly, even in the case where the antibody to be screened is not a low-molecular antibody to be synthesized, a low-molecular antibody can be designed by grafting the CDR regions of the antibody into the FR regions of the low-molecular antibody to be synthesized.

### 4. Multimerization of low-molecular antibody of the present invention

The low-molecular antibody of the present invention may be linked by simple peptide bonds or linkers and multimerized into a polyvalent antibody or multi-specific antibody. Such a polyvalent antibody or multi-specific antibody falls within the scope of the present invention.

For example, scFvs can be linked by peptide bonds to produce (scFv) ₂. Further, scFVs can be dimerized to form diabodies, trimerized to form triabodies, and tetramerized to form tetra bodies. Further, it is also possible to produce more complex tandem diabodies or flexibodies. Likewise, a single-domain antibody such as VHH can also be linked in tandem or multimerized with simple peptide bonds or linkers. The polyvalent antibody thus constructed can be made into a multi-specific antibody having binding properties to different target proteins by making the variable domains of the constituent monomers different.

As a linker, a peptide linker composed of amino acids can be used, for example, a linker composed of Gly and Ser can be used. The length of the peptide linker is 1 to 50 amino acids, preferably 1 to 30 amino acids, more preferably 1 to 10 amino acids.

Alternatively, they may be linked using a chemical crosslinking agent (synthetic chemical linker). Examples of the available chemical crosslinking agent can include NHS, DSS, BS3, DSP, DTSSP, EGS, sulfo-EGS, DST, sulfo-DST, BSOCOES, and sulfo-BSOCOES.

### 5. Modification of low-molecular antibody of the present invention

The low-molecular antibody of the present invention can be appropriately modified depending on the purpose of use. Examples of modification can include modifications by fluorescent labels, phosphorescent labels, chemiluminescent labels, bioluminescent labels, radioisotopes, metals, metal chelates, metal cations, chromophores, enzymes, lipids, hydrophilic polymers, and carbohydrates.

Modifications include modifications for introducing linkers and spacers for conjugation with drugs, and chemical modifications for stabilization. Accordingly, one or several amino acids may be substituted with nonnatural amino acids other than D-amino acids or L-amino acids as long as the specificity and three-dimensional structure of the low-molecular antibody are not adversely affected.

Further, the low-molecular antibody of the present invention may be immobilized on a suitable surface depending on the purpose of use. Examples of carriers for immobilization include colloidal particles, magnetic particles, and microplates.

### 6. Use of low-molecular antibody

### 6.1 Therapeutic use

It is conceivable that, since proteins composed of D-amino acids are difficult to undergo substrate recognition by in vivo peptidases, antigen presentation resulting from fragmentation in antigen-presenting cells (APC) is unlikely to occur. Therefore, as compared with natural-type (L-) low-molecular antibodies, mirror-image (D-) low-molecular antibodies are expected to be less susceptible to T-cell activation due to antigen presentation and to suppress differentiation of B cells due to the activated T cells to reduce the risk of producing anti-drug antibodies (ADAs). In this way, the low-molecular antibody of the present invention, which have specificities similar to those of natural antibody molecules but have reduced immunogenicity, can be used as pharmaceuticals either alone or in combination with other drugs.

### "Pharmaceutical composition"

In the case where the low-molecular antibody of the present invention binds to a target protein to exert a predetermined pharmacological action, the low-molecular antibody can be formulated with pharmacologically acceptable carriers and additives to be used as a pharmaceutical composition.

Examples of the pharmacologically acceptable carriers and additives include surfactants, excipients, colorants, flavors, preservatives, antioxidants, stabilizers, buffers, suspending agents, isotonizing agents, binders, disintegrants, lubricants, fluidity enhancers, and flavoring agents, but there is no limitation to these examples.

Specifically, aqueous carriers include water, ethanol, polyols (such as glycerol, propylene glycol, and polyethylene glycol), vegetable oils such as olive oil, and organic esters such as ethyl oleate.

Examples of non-aqueous carriers can include light anhydrous silicic acid, lactose, crystalline cellulose, mannitol, starch, carmellose calcium, carmellose sodium, hydroxypropylcellulose, hydroxypropylmethylcellulose, polyvinyl acetal diethylaminoacetate, polyvinylpyrrolidone, gelatin, medium-chain fatty acid triglyceride, polyoxyethylene hardened castor oil 60, white sugar, carboxymethylcellulose, corn starch, and inorganic salts.

### "Antibody drug conjugate (ADC)"

Due to the specific target recognition function of the antibody, the low-molecular antibody of the present invention can be used as an antibody drug conjugate (ADC) by being conjugated with an appropriate drug via a linker.

For example, an anticancer agent can act specifically in a tumor cell by producing a low-molecular antibody that binds to a tumor cell-specific antigen, conjugating it with the anticancer agent, and designing it so that the linker cleaves in the tumor cells (internalized ADC). Further, an anticancer agent can act in the vicinity of a tumor cell by using a linker that cleaves in the tumor microenvironment outside the tumor cells (exogenous ADC). In some cases, non-cleavable linkers can also be used. Cleavable linkers can be designed, for example, based on disulfides, hydrazones, and peptides, and non-cleavable linkers can be designed based on thioethers or the like (non-cleavable). Linkers can be appropriately designed according to the purpose, and such techniques are also known in the art.

An ADC containing the low-molecular antibody of the present invention can be formulated with pharmacologically acceptable carriers and additives, and such carriers and additives are as described above.

The administration route of the pharmaceutical composition containing the low-molecular antibody of the present invention or ADC is not specifically limited, but parenteral administration is preferable, and specific examples include injection administration, nasal administration, pulmonary administration, and transdermal administration. Examples of the injection administration include intravenous injection, intramuscular injection, intraperitoneal injection, and subcutaneous injection. The administration method can be appropriately selected depending on the patient's age and symptoms.

The dose of the pharmaceutical composition containing the low-molecular antibody of the present invention or ADC is adjusted to bring about an optimal response (for example, therapeutic response) depending on the intended therapeutic effect, administration method, treatment period, and the patient's age and body weight, but is generally 10 µg/kg to 10 mg/kg as active components per day for an adult. As typical treatment methods, administration once a week, administration once every two weeks, administration once every three weeks, administration once every four weeks, administration once a month, administration once every three months, or administration once every 3 to 6 months can be mentioned, for example.

### 6.2 Diagnostic use

The low-molecular antibody of the present invention has high stability in vivo and low immunogenicity and thus can be used for diagnosis or imaging by being modified with a detectable molecule.

Examples of the detectable molecule can include radionuclides: technetium 99 m (^{99m}Tc) , iodine 123 (¹²³I), iodine 125 (¹²⁵I), indium 111 (¹¹¹In) , fluorine 18 (¹⁸F) , gallium 67 (⁶⁷Ga), gallium 68 (⁶⁸Ga), and copper 64 (⁶⁴Cu); fluorescent molecules: fluorescein, Alexa, and cyanine; chemiluminescence molecules: luminols; bioluminescent molecules: and luciferase and alkaline phosphatase.

The low-molecular antibody modified with detectable molecules can be detected by radioimmunoassay (RIA), enzyme immunoassay (EIA), fluorescence immunoassay (FIA), luminescence immunoassay (LIA), immunoprecipitation (IP), immunonephelometry (TIA), Western blot (WB), immunohistochemistry (IHC), immunity diffuse method (SRID) according to the modifier molecules.

### 7. Kit for producing low-molecular antibody

In a VHH antibody, CDR3 is the richest in diversity in the three CDR regions and is important for interaction with the target, and the other two CDRs are considered to have a small effect on interaction and can be fixed sequences. Accordingly, a segment before cysteine (Cys⁹⁶) in the FR3 region (D-9 in Examples described below) or a partial segment constituting the segment is prepared in advance, to provide a kit for producing a VHH antibody. The remaining segments are designed and synthesized according to the target, and ligated to the segment of the kit by the aforementioned NCL method, to produce a target-specific VHH antibody.

For example, the kit of the present invention contains one or a plurality of polypeptides including the following amino acid sequence (a) or (b) and composed of D-amino acids and achiral glycine:
(a) an amino acid sequence set forth in any one of SEQ ID NOs: 55 to 57, 61, and 63; or
(b) an amino acid sequence at least having an identity of 60, 70%, 80%, 90%, 95%, 98%, or 99% with the amino acid sequence set forth in any one of SEQ ID NOs: 55 to 57, 61, and 63 (preferably in the portion corresponding to the framework regions of the sequence).

Examples of the amino acid sequence (b) include the sequence of the portion corresponding to the framework regions substituted with the sequence corresponding to the framework regions of another known VHH antibody (for example, see SEQ ID NOs: 5 to 20 or SEQ ID NOs: 21 to 54 (Figure 8)).

Preferably, the kit of the present invention includes:
(1) a polypeptide containing the amino acid sequence set forth in SEQ ID NO: 63 (D-9 in Examples) that is the segment before cysteine (Cys⁹⁶) in the FR3 region and composed of D-amino acids and achiral glycine; or
(2) a polypeptide containing the amino acid sequence set forth in SEQ ID NO: 55 (D-1 in Examples) that is a part of the FR1 region excluding the CDR regions and composed of D-amino acids and achiral glycine, or
a polypeptide containing amino acid sequences at least having an identity of 60, 70%, 80%, 90%, 95%, 98%, or 99% with the aforementioned amino acid sequences preferably in a portion corresponding to the framework regions.

Each polypeptide may be appropriately modified with a protective group, a label such as a histidine tag or a fluorescent label, or may be immobilized on a solid phase such as beads or a support. For modification and immobilization, see the description in 5.

The kit may contain reagents and buffers necessary for synthesis, D-amino acids, achiral glycine, instructions for use, etc., in addition to the above polypeptides, which are essential components.

### Examples

Hereinafter, the present invention will be described specifically by way of examples, but the present invention is not limited to these examples.

### Example 1

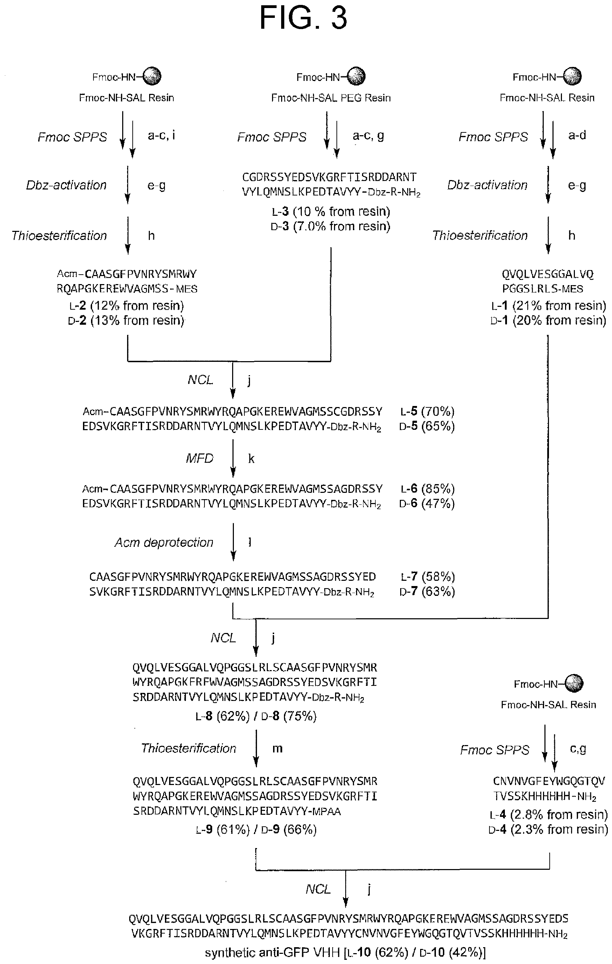

In this example, a GFP-bound VHH antibody (L-GFP-VHH antibody) and its enantiomer (D-GFP-VHH antibody) were synthesized by the process shown in Figure 3.

### (1) HPLC and MS

Analytical high-performance liquid chromatography (HPLC) was performed using a COSMOSIL 5C18-AR300 column (4.6 × 250 mm, available from NACALAI TESQUE, INC.) at a flow rate of 1 mL/min (25°C) with a linear gradient of 0.1% (v/v) TFA-containing CH₃CN. L-10 and D-10 were analyzed using a COSMOSIL 5C4-AR300 column (4.6 × 150 mm, available from NACALAI TESQUE, INC.) at a flow rate of 1 mL/min (40°C) with a linear gradient of 0.1% (v/v) TFA-containing CH₃CN. The products were detected at an absorbance of 220 nm.

For HPLC purification, a COSMOSIL 5C18-AR300 column (20 × 250 mm, available from NACALAI TESQUE, INC.) or a COSMOSIL 5C4-AR300 column (20 × 150 mm, available from NACALAI TESQUE, INC.) was used at a flow rate of 8 mL/min (at room temperature) with a linear gradient of 0.1% (v/v) TFA-containing CH₃CN. For L-10 and D-10, a COSMOSIL 5C4-AR300 column (20 × 150 mm, available from NACALAI TESQUE, INC.) was used at a flow rate of 12 mL/min (40°C) with a linear gradient of 0.1% (v/v) TFA-containing CH₃CN.

### (2) Fmoc-SPPS

Solid-phase peptide synthesis by the Fmoc method (Fmoc-SPPS) was performed using an automated peptide synthesizer (PSSM-8, available from SHIMADZU CORPORATION). The following side-chain-protected amino acids were used: Arg(Pbf), Asn(Trt), Asp(OtBu), Cys(Trt), Gln(Trt), Glu(OtBu), His(Trt), Lys(Boc), Ser(tBu), Thr(tBu), Tyr(tBu). Using HBTU (5 equivalent amount), HOBt-H₂O (5 equivalent amount), and (i-Pr)₂NEt (10 equivalent amount), coupling of Fmoc-protected amino acid (5 equivalent amount) was performed twice in DMF for 45 minutes. The Fmoc protecting group was deprotected twice with 20% piperidine/DMF for 4 minutes.

### (3) Synthesis of L-1

A peptide sequence was constructed from a Fmoc-NH-SAL resin (45 mg, 0.02 mmol) by the standard procedure for Fmoc-SPPS described above. After constructing the peptide chains, it was reacted with Boc₂O (22 mg, 0.10 mmol) and (i-Pr)₂NEt (35 µL, 0.20 mmol) in DMF for 2 hours, to protect amine at the N-terminus. After washing, the resin was treated with 50 mM 4-nitrophenyl chloroformate in DCM (2 mL) for 1 hour and then treated with 0.5M (i-Pr)₂NEt in DMF (2 mL) for 15 minutes. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. The precipitate obtained was dissolved in a minimum amount of MESNa buffer (500 mM MESNa, 6 M guanidine, 200 mM phosphate buffer: pH 6.0) and incubated for 1 hour. The crude product was purified by HPLC, to obtain a desired peptide L-1 (9.1 mg, yield: 21%, SEQ ID NO: 55).
MS (ESI) : calculated for C₉₂H₁₅₉N₂₇O₃₂S₂: 2219.56; measured (m/z) : [M+2H]²⁺ = 1110.60.

### (4) Synthesis of L-2

A protective peptide was constructed from a Fmoc-NH-SAL resin (45 mg, 0.02 mmol) by the standard procedure for Fmoc-SPPS described above. For coupling of the N-terminal cysteine of peptide L-2, Boc-Cys(Acm)-OH was used together with HBTU (38 mg, 0.10 mmol), HOBt-H₂O (15 mg, 0.10 mmol), and (i-Pr)₂NEt (35 µL, 0.20 mmol). After washing, the resin was treated with 50 mM 4-nitrophenyl chloroformate in DCM (2 mL) for 1 hour and then treated with 0.5-M (i-Pr)₂NEt in DMF (2 mL) for 15 minutes. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. The precipitate obtained was dissolved in a minimum amount of MESNa buffer (500 mM MESNa, 6 M guanidine, 200 mM phosphate buffer: pH 6.0) and incubated for 1 hour. The crude product was purified by HPLC, to obtain a desired peptide L-2 (9.3 mg, yield: 12%, SEQ ID NO: 56).
MS (ESI) : calculated for C₁₆₆H₂₅₀N₅₀O₄₈S₅: 3874.43; measured (m/z): [M+4H]⁴⁺ = 969.56, [M+3H]³⁺ = 1292.52, [M+2H]²⁺ = 1937.87.

### (5) Synthesis of L-3

A protective peptide was constructed from a Fmoc-NH-SALPEG resin (87 mg, 0.02 mmol) by the standard procedure for Fmoc-SPPS described above. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. The precipitate obtained was dissolved in a minimum amount of a 0.1% TFA-containing 50% CH₃CN solution. The crude product was purified by HPLC, to obtain a desired peptide L-3 (11 mg, yield: 10%, SEQ ID NO: 57).
MS (ESI) : calculated for C₂₂₀H₃₄₀N₆₆O₇₃S₂: 5141.65; measured (m/z): [M+6H]⁶⁺ = 858.17, [M+5H]⁵⁺ = 1029.63, [M+4H]⁴⁺ = 1286.57, [M+3H]³⁺ = 1714.78.

### (6) Synthesis of L-4

A protective peptide was constructed from a Fmoc-NH-SAL resin (93 mg, 0.04 mmol) by the standard procedure for Fmoc-SPPS described above. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. The precipitate obtained was dissolved in a minimum amount of a 0.1% TFA-containing 50% CH₃CN solution. The crude product was purified by HPLC, to obtain a desired peptide L-4 (3.6 mg, yield: 2.8%, SEQ ID NO: 58).
MS (ESI) : calculated for C₁₃₇H₁₉₄N₄₆O₃₈S: 3125.40; measured (m/z): [M+4H]⁴⁺ = 782.59, [M+3H]³⁺ = 1043.02, [M+2H]²⁺ = 1564.07.

### (7) Synthesis of L-5

Thioester L-2 (7.7 mg, 2.0 µmol) and peptide L-3 (11 mg, 2.1 µmol) were reacted in a ligation solution (400 mM MPAA, 100 mM TCEP, 6 M guanidine, 200 mM phosphate buffer: pH 7.0; 1.0 mL) at 37°C for 2 hours. The reaction was monitored by analytical HPLC. The crude product was purified by HPLC, to obtain a desired peptide L-5 (12 mg, 70% yield: SEQ ID NO: 59).
MS (ESI): calculated for C₃₈₄H₅₈₄N₁₁₆O₁₁₈S₅: 8873.89; measured (m/z): [M+11H]¹¹⁺ = 808.22, [M+10H]¹⁰⁺ = 888.67, [M+9H]⁹⁺ = 987.29, [M+8H]⁸⁺ = 1110.53, [M+7H]⁷⁺ = 1268.91, [M+6H]⁶⁺ = 1480.28, [M+5H]⁵⁺ = 1775.91.

### (8) Synthesis of L-6

Peptide L-5 (13 mg, 1.5 µmol) was treated with a desulfurization solution (20 mM VA-044, 100 mM MESNa, 250 mM TCEP, 6 M guanidine, 100 mM phosphate buffer: pH 6.5;3.0 mL) at 37°C for 2 hours. The reaction was monitored by analytical HPLC. The crude product was purified by HPLC, to obtain a desired peptide L-6 (11 mg, 85% yield: SEQ ID NO: 60).
MS (ESI): calculated for C₃₈₄H₅₈₄N₁₁₆O₁₁₈S₄: 8841.83; measured (m/z): [M+8H]⁸⁺ = 1106.64, [M+7H]⁷⁺ = 1264.47, [M+6H]⁶⁺ = 1474.84, [M+5H]⁵⁺ = 1769.45.

### (9) Synthesis of L-7

Peptide L-6 (6.9 mg, 0.78 µmol) and PdCl₂ (1.4 mg, 7.8 µmol) were reacted in a 100 mM phosphate buffer containing 6 M guanidine (390 µL) at 37°C for 30 minutes. Then, a small amount of DTT was added. The reaction was monitored by analytical HPLC. The crude product was purified by HPLC, to obtain a desired peptide L-7 (3.9 mg, yield: 58%, SEQ ID NO: 61).
MS (ESI): calculated for C₃₈₁H₅₇₉N₁₁₅O₁₁₇S₄: 8770.75; measured (m/z): [M+11H]¹¹⁺ = 798.56, [M+10H]¹⁰⁺ = 878.51, [M+9H]⁹⁺ = 975.89, [M+8H]⁸⁺ = 1097.46, [M+7H]⁷⁺ = 1254.52, [M+6H]⁶⁺ = 1462.83, [M+5H]⁵⁺ = 1755.07.

### (10) Synthesis of L-8

Thioester L-1 (1.8 mg, 0.80 µmol) and peptide L-7 (2.3 mg, 0.27 µmol) were reacted in a ligation buffer (400 mM MPAA, 100 mM TCEP, 6 M guanidine, 200 mM phosphate buffer: pH 7.0; 0.14 mL) at 37°C for 1 hour. The reaction was monitored by analytical HPLC. The crude product was purified by HPLC, to obtain a desired peptide L-8 (1.8 mg, yield: 62%, SEQ ID NO: 62).
MS (ESI): calculated for C₄₇₁H₇₃₂N₁₄₂O₁₄₆S₄: 10848.13; measured (m/z): [M+13H]¹³⁺ = 835.72, [M+12H]¹²⁺ = 905.33, [M+11H]¹¹⁺ = 987.56, [M+10H]¹⁰⁺ = 1086.31, [M+9H]⁹⁺ = 1206.72, [M+8H]⁸⁺ = 1357.45, [M+7H]⁷⁺ = 1551.12, [M+6H]⁶⁺ = 1809.18.

### (11) Synthesis of L-9

Peptide L-8 (1.1 mg, 0.10 µmol) was activated in an activation solution (133 mM NaNO₂, 6 M guanidine, 200 mM phosphate buffer: pH 3.0; 37.5 µL) at -20°C for 30 minutes. A ligation solution (400 mM MPAA, 100 mM TCEP, 6-M guanidine, 200 mM phosphate buffer: pH 7.0; 12.5 µL) was added thereto, to provide the thioester. The reaction was monitored by analytical HPLC. The crude product was purified by HPLC, to obtain a desired peptide L-9 (0.66 mg, yield: 61%, SEQ ID NO: 63).
MS (ESI): calculated for C₄₆₆H₇₁₉N₁₃₅O₁₄₆S₅: 10708.98; measured (m/z): [M+12H]¹²⁺ = 893.56, [M+11H]¹¹⁺ = 974.98, [M+10H]¹⁰⁺ = 1072.25, [M+9H]⁹⁺ = 1191.34, [M+8H]⁸⁺ = 1339.96, [M+7H]⁷⁺ = 1530.98, [M+6H]⁶⁺ = 1785.87.

### (12) Synthesis of L-10 (synthetic L-GFP-VHH antibody)

Thioester L-9 (7.6 mg, 0.71 µmol) and peptide L-4 (5.2 mg, 1.7 µmol) were reacted in a ligation solution (400 mM MPAA, 100 mM TCEP, 6 M guanidine, 200 mM phosphate buffer: pH 7.0; 1.1 mL) at room temperature for 10 hours. The reaction was monitored by analytical HPLC. The crude product was purified by HPLC, to obtain a desired peptide L-10 (6.0 mg, yield: 62%, SEQ ID NO: 64). MS (ESI): calculated for C₅₉₅H₉₀₅N₁₈₁O₁₈₂S₅: 13666.17; measured (m/z): [M+14H]¹⁴⁺ = 977.81, [M+13H]¹³⁺ = 1052.81, [M+12H]¹²⁺ = 1140.62, [M+10H]¹⁰⁺ = 1368.11, [M+9H]⁹⁺ = 1519.85, [M+8H]⁸⁺ = 1709.45, [M+7H]⁷⁺ = 1954.09.

### (13) Synthesis of D-1

D-1 was obtained by the same process as in the synthesis of L-1 described above (60.9 mg, yield: 20%, SEQ ID NO: 55 (all amino acids are D-forms except achiral glycine; the same applies to the following description)). MS (ESI) : calculated for C₉₂H₁₅₉N₂₇O₃₂S₂: 2219.56; measured (m/z) : [M+2H]²⁺ = 1110.97.

### (14) Synthesis of D-2

D-2 was obtained by the same process as in the synthesis of L-2 described above (68.6 mg, yield: 13%, SEQ ID NO: 56).
MS (ESI) : calculated for C₁₆₆H₂₅₀N₅₀O₄₈S₅: 3874.43; measured (m/z): [M+4H]⁴⁺ = 969.87, [M+3H]³⁺ = 1292.76, [M+2H]²⁺ = 1938.16.

### (15) Synthesis of D-3

D-3 was obtained by the same process as in the synthesis of L-3 described above (7.2 mg, yield: 7.0%, SEQ ID NO: 57).
MS (ESI) : calculated for C₂₂₀H₃₄₀N₆₆O₇₃S₂: 5141.65; measured (m/z): [M+6H]⁶⁺ = 858.33, [M+5H]⁵⁺ = 1029.56, [M+4H]⁴⁺ = 1286.77, [M+3H]³⁺ = 1715.34.

### (16) Synthesis of D-4

D-4 was obtained by the same process as in the synthesis of L-4 described above (10.2 mg, yield: 2.3%, SEQ ID NO: 58).
MS (ESI) : calculated for C₁₃₇H₁₉₄N₄₆O₃₈S: 3125.40; measured (m/z): [M+4H]⁴⁺ = 782.66, [M+3H]³⁺ = 1043.10, [M+2H]²⁺ = 1563.31.

### (17) Synthesis of D-5

D-5 was obtained by the same process as in the synthesis of L-5 described above (13.8 mg, yield: 65%, SEQ ID NO: 59).
MS (ESI): calculated for C₃₈₄H₅₈₄N₁₁₆O₁₁₈S₅: 8873.89; measured (m/z): [M+11H]¹¹⁺ = 808.08, [M+10H]¹⁰⁺ = 888.83, [M+9H]⁹⁺ = 987.60, [M+8H]⁸⁺ = 1110.83, [M+7H]⁷⁺ = 1269.51, [M+6H]⁶⁺ = 1480.21, [M+5H]⁵⁺ = 1776.25.

### (18) Synthesis of D-6

D-6 was obtained by the same process as in the synthesis of L-6 described above (3.7 mg, yield: 47%, SEQ ID NO: 60).
MS (ESI): calculated for C₃₈₄H₅₈₄N₁₁₆O₁₁₈S₄: 8841.83; measured (m/z): [M+8H]⁸⁺ = 1106.65, [M+7H]⁷⁺ = 1264.88, [M+6H]⁶⁺ = 1474.73, [M+5H]⁵⁺ = 1769.45.

### (19) Synthesis of D-7

D-7 was obtained by the same process as in the synthesis of L-7 described above (3.1 mg, yield: 63%, SEQ ID NO: 61).
MS (ESI): calculated for C₃₈₁H₅₇₉N₁₁₅O₁₁₇S₄: 8770.75; measured (m/z): [M+11H]¹¹⁺ = 798.58, [M+10H]¹⁰⁺ = 878.33, [M+9H]⁹⁺ = 975.88, [M+8H]⁸⁺ = 1097.92, [M+7H]⁷⁺ = 1254.38, [M+6H]⁶⁺ = 1463.04, [M+5H]⁵⁺ = 1755.13.

### (20) Synthesis of D-8

D-8 was obtained by the same process as in the synthesis of L-8 described above (3.6 mg, yield: 75%, SEQ ID NO: 62).
MS (ESI): calculated for C₄₇₁H₇₃₂N₁₄₂O₁₄₆S₄: 10848.13; measured (m/z): [M+13H]¹³⁺ = 835.71, [M+12H]¹²⁺ = 905.52, [M+11H]¹¹⁺ = 987.80, [M+10H]¹⁰⁺ = 1086.24, [M+9H]⁹⁺ = 1207.04, [M+8H]⁸⁺ = 1357.72, [M+7H]⁷⁺ = 1551.36, [M+6H]⁶⁺ = 1809.89.

### (21) Synthesis of D-9

D-9 was obtained by the same process as in the synthesis of L-9 described above (7.1 mg, yield: 66%, SEQ ID NO: 63).
MS (ESI): calculated for C₄₆₆H₇₁₉N₁₃₅O₁₄₆S₅: 10708.98; measured (m/z): [M+11H]¹¹⁺ = 975.07, [M+10H]¹⁰⁺ = 1072.56, [M+9H]⁹⁺ = 1191.18, [M+8H]⁸⁺ = 1340.21, [M+7H]⁷⁺ = 1531.30, [M+6H]⁶⁺ = 1786.13.

### (22) Synthesis of D-10 (synthetic D-GFP-VHH antibody)

D-10 was obtained by the same process as in the synthesis of L-10 described above (3.8 mg, yield: 42%, SEQ ID NO: 64).
MS (ESI): calculated for C₅₉₅H₉₀₅N₁₈₁O₁₈₂S₅: 13666.17; measured (m/z): [M+14H]¹⁴⁺ = 977.48, [M+13H]¹³⁺ = 1052.68, [M+12H]¹²⁺ = 1140.47, [M+9H]⁹⁺ = 1519.70, [M+8H]⁸⁺ = 1710.32, [M+7H]⁷⁺ = 1953.53.

### (23) Preparation of recombinant protein

A recombinant L-GFP-VHH antibody plasmid was prepared by cloning the C-terminal 6 × His into pGEX6P1-GFP-Nanobody (Addgene#61838). All plasmids were transformed into BL21 (DE3) cells and cultured in LB medium. E. coli was cultured overnight at 37°C in LB medium containing ampicillin (final concentration: 50 µg/mL), and 100 mM IPTG was added to express overnight at 20°C. E. coli was centrifuged (at 6,000 rpm and 4°C for 20 minutes) and then resuspended in a ligation buffer (5 mM DTT and protease inhibitor in PBS [available from NACALAI TESQUE, INC.]). After sonication and addition of TritionX-100, cell lysates were centrifuged at 4°C and 12,000 rpm for 30 minutes. The supernatant was incubated overnight at 4°C with glutathione-Sepharose 4B (GE Healthcare). For purification of the recombinant L-GFP-VHH antibody, the GST tag was removed by adding PreScission Protease (GEHelthcare) in a cleavage buffer (50 mM Tris-HCl: pH 7.5,150 mM NaCl,1 mM EDTA,1 mM DTT, 0.01% TritonX-100) and incubating the mixture overnight at 4°C. For purification of GST-EGFP and GST-mCherry, the GST-tagged protein was eluted with an elution buffer (PBS: pH 8.5 containing 10 mM glutathione) at 4°C for 30 minutes. The GST-mCherry protein was further purified by gel filtration chromatography (Superdex75 Increase, GE Healthcare) using PBS. All protein purities were confirmed by SDS-PAGE.

### (24) Folding of synthetic L-GFP-VHH antibody and synthetic D-GFP-VHH antibody

Peptide L-10 or D-10 was dissolved in PBS (pH 7.4) containing 6M guanidine and 40 mM DTT at 1.0 mg/mL and incubated at room temperature for 2 hours. This solution was diluted 100-fold with PBS (pH 7.4) and allowed to stand overnight at room temperature. The solution was concentrated using MWCO3000 centrifugal filtration membrane (Millipore, Amicon-Ultra3kDa).

### (25) CD spectra of recombinant L-GFP-VHH antibody, synthetic L-GFP-VHH antibody, and synthetic D-GFP-VHH antibody

Recombinant L-GFP-VHH antibody, synthetic L-GFP-VHH antibody, and synthetic D-GFP-VHH antibody were diluted with PBS (pH 7.4) and adjusted to a concentration of 10 µM each. CD spectra of each protein were recorded at 20°C using a circular dichroism spectrometer (JACSO J-720). The thermostability of each protein was estimated by measuring changes between 37°C and 95°C at a wavelength of 203 nm.

The CD spectrum of the synthetic L-GFP-VHH antibody was consistent with that of the recombinant L-GFP-VHH antibody, suggesting the presence of β-sheet structure (Figure 4A). The CD spectrum of the synthetic D-GFP-VHH antibody was inverse to that of the recombinant L-GFP-VHH antibody. The synthetic L- and D-GFP-VHH antibodies had the same thermostability as the recombinant L-GFP-VHH antibody. D-VHH antibody scaffolds were suggested to be stable in physiological conditions (Figure 4B). These results indicate that both synthetic L- and D-GFP-VHH antibodies were correctly folded.

### (26) Enzyme-linked immunosorbent assay (ELISA)

ELISA was performed in PBS (pH 7.4) containing 0.025% Tween20 for all washing and dilution processes. 96-well microtiter plates (Greiner, high binding) were coated with GST-EGFP or GST-mCherry in 50 mM sodium carbonate buffer (pH 9.4) overnight at 4°C (50 µL/well; 30 nM). After coating, the wells were washed three times and blocked with PBS containing 3% BSA (150 µL/well) for 2 hours. After washing three times, recombinant or synthetic L-GFP-VHH antibody (50 µL/well, 0, 0.03, 0.1, 0.3, 1, 3, 10, 30 nM) was added and incubated for 1 hour. After washing three times, anti-histidine monoclonal antibody (28-75) (Wako) (50 µL/well) was added and incubated at 4°C for 1 hour. After washing three times, anti-mouse IgG (H+L) HRP conjugate (Promega) (50 µL/well) was added and incubated at 4°C for 1 hour. After washing five times, TMB solution was added and incubated for 15 minutes, to stop the reaction using 1 M H₂SO₄ (50 µL/well). The absorbance was measured at 450 nm using an iMark Microplate Absorbance Reader (Bio-Rad).

Both recombinant and synthetic L-GFP-VHH antibodies bind to GST-EGFP but not to GST-mCherry, which suggests that synthetic L-GFP-VHH antibody has similar biological activity to recombinant L-GFP-VHH antibody (Figure 5).

### (27) Quartz crystal microbalance (QCM) analysis

QCM analysis was performed using Single-Q (SCINICS, Japan). The gold surface of the sensor chip was washed with piranha solution, and 0.2 mM NTA-SAM formation reagent (Dojindo, Japan) dissolved in 10% EtOH was added and incubated overnight. After washing, 40 mM NiSO₄ solution was added and incubated for 1 hour. After washing and equilibrating with 500 µL of PBS (pH 7.4), 5 µL of 1.0-mg/mL ligand solution (recombinant L-GFP-VHH antibody, synthetic L-GFP-VHH antibody, or synthetic D-GFP-VHH antibody in PBS) was added. After washing and equilibrating with 500 µL of PBS (pH 7.4), each sample solution (final concentration; 0.1 nM, 0.3 nM, 1 nM, 3 nM, 10 nM, 30 nM, 100 nM, and 300 nM) was added repeatedly. The dissociation constant was evaluated in triplicate assays.

Recombinant and synthetic L-GFP-VHH antibodies interacted with GST-EGFP, but no binding to GST-mCherry was observed (Figure 6). The synthetic D-GFP-VHH antibody was also immobilized on the Ni-NTA-SAM surface, but no interaction between the synthetic D-GFP-VHH antibody and GST-EGFP was observed.

These results confirmed the GFP-binding activity of the synthetic L-GFP-VHH antibody. Further, it was also found that the synthetic D-GFP-VHH antibody had a perfect mirror image structure of the L-GFP-VHH antibody and had a different interaction surface.

### (28) Immunization and immunogenicity assays

Six BALB/c mice (female, 6 weeks old) per group were injected intraperitoneally with synthetic L- or D-GFP-VHH antibody antigen emulsified in complete Freund's adjuvant (Wako) at 50 µg/injection on day 0. Synthetic L- or D-GFP-VHH antibody antigen emulsified in incomplete Freund's adjuvant (Wako) was injected on days 14 and 21 at 50 µg/injection. Immune sera were collected on days 0, 14, 21 and 28. ELISA was performed using PBS (pH 7.4) containing 0.025% Tween20 in all washing and dilution processes. 96-well microtiter plates (Greiner, high binding) were coated overnight at 4°C either with L-GFP-VHH antibody or D-GFP-VHH antibody (50 µL/well; 1.0 ng/mL, 10 ng/mL, 100 ng/mL, or 1,000 ng/mL) in 50 mM sodium carbonate buffer (pH 9.4). After coating, the wells were washed three times and blocked with PBS containing 3% BSA (150 µL/well) for 2 hours. After washing three times, a 1:1,000 dilution of each mouse's immunized serum was added (50 µL/well, days 0, 14, 21, and 28) and incubated for 1 hour. After washing three times, a 1:5,000 dilution of Anti-Mouse IgG (H+L) HRP Conjugate (Promega) (50 µL/well) was added and incubated for 30 minutes. After washing four times, TMB solution was added and incubated for 15 minutes, to stop the reaction using 1 M H₂SO₄ (50 µL/well). The absorbance was measured at 450 nm using an iMark Microplate Absorbance Reader (Bio-Rad).

The presence of antibodies to L-GFP-VHH antibody was observed in sera of all mice immunized with L-GFP-VHH antibody (14 days after immunization) (Figure 7). The concentration of ADA increased with repeated injections depending on the antigen coating concentration. In contrast, the presence of antibodies against D-GFP-VHH antibody was not observed in the sera of all mice immunized with D-GFP-VHH antibody even after 28 days. No appearance of ADA was observed when mice were immunized with the opposite enantiomeric form of the antigen. These results suggest that the D-GFP-VHH antibody is nonimmunogenic, whereas the L-GFP-VHH antibody is immunogenic.

Although the above example was performed using the anti-GFP VHH antibody, those skilled in the art will appreciate that these results are applicable to other target proteins.

### Example 2

In this example, the VHH antibody PMP12A2h1 and its enantiomer (D-PMP12A2h1) were synthesized by the process shown in Figure 9. PMP12A2h1 is a VHH antibody constituting a drug known as the generic name of caplacizumab, specifically binds to the A1 domain (vWF A1 domain) of von Willebrand factor that is one of the blood coagulation factors, and suppresses platelet aggregation.

### (1) HPLC and MS

Analytical high-performance liquid chromatography (HPLC) was performed using a COSMOSIL 5C18-AR300 column (4.6 × 250 mm, available from NACALAI TESQUE, INC.) at a flow rate of 1 mL/min (25°C) with a linear gradient of 0.1% (v/v) TFA-containing CH₃CN. The products were detected at an absorbance of 220 nm.

For HPLC purification, a COSMOSIL 5C18-AR300 column (20 × 250 mm, available from NACALAI TESQUE, INC.) or COSMOSIL 5C4-AR300 column (20 × 150 mm, available from NACALAI TESQUE, INC.) was used at a flow rate of 8 mL/min (at room temperature) with a linear gradient of 0.1% (v/v) TFA-containing CH₃CN.

### (2) Construction of protective peptide resin

Solid-phase peptide synthesis (Fmoc-SPPS) by the Fmoc method was performed using an automated peptide synthesizer (PSSM-8, available from SHIMADZU CORPORATION or Liberty BLUE, CEM). In the synthesis using PSSM-8, the following side-chain-protected amino acids were used: Arg (Pbf), Asn (Trt), Asp (OtBu), Cys (Trt), Gln (Trt), Glu (OtBu), His (Trt), Lys (Boc), Ser(tBu), Thr (tBu), and Tyr (tBu). Using Oxyma Pure (5 equivalent amount) and DIC (10 equivalent amount), coupling of Fmoc-protected amino acid (5 equivalent amount) was performed twice in DMF for 60 minutes. The Fmoc protecting group was deprotected twice with 20% piperidine/DMF for 4 minutes.

### (3) Construction of protective peptide resin with microwave irradiation

In peptide synthesis (Liberty BLUE) with microwave irradiation, the following side-chain-protected amino acids were used: Arg(Pbf), Asn(Trt), Asp(OtBu), Cys(Trt), Gln(Trt), Glu(OtBu), His (Trt), Lys(Boc), Ser(tBu), Thr (tBu), Trp(Boc), Tyr(tBu). Using Oxyma Pure (5 equivalent amount) and DIC (10 equivalent amount), coupling of Fmoc-protected amino acid (5 equivalent amount) was performed in DMF under microwave irradiation at 90°C for 125 seconds. For Arg(Pbf), coupling was performed twice in DMF under microwave irradiation at 90°C for 125 seconds. For His(Trt), coupling was performed twice in DMF under microwave irradiation at 50°C for 10 minutes. The Fmoc protecting group was deprotected in 20% piperidine/DMF under microwave irradiation at 90°C for 90 seconds.

### (4) Synthesis of L-11

A peptide sequence was constructed from a Fmoc-NH-SAL resin (439 mg, 0.25 mmol) by the standard procedure for Fmoc-SPPS described above. After condensation of Fmoc-Arg(Pbf)-OH, Fmoc-Dbz-OH, and Fmoc-Ser(tBu)-OH in this order, the peptide chain was elongated by microwave-assisted Fmoc-SPPS. After constructing the peptide chain, it was reacted with Boc₂O (273 mg, 1.25 mmol) and (i-Pr)₂NEt (435 µL, 2.5 mmol) in DMF for 2 hours, to protect the N-terminal amine. After washing, the resin was treated with 50 mM 4-nitrophenyl chloroformate in DCM (25 mL) for 1 hour and then was treated with 0.5 M (i-Pr)₂NEt in DMF (25 mL) for 15 minutes. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. After the precipitate obtained was dissolved in a 0.1% TFA-containing 50% CH₃CN solution, the crude product was purified by HPLC, to obtain a desired peptide L-11 (113 mg, yield: 19%, SEQ ID NO: 65).
MS (ESI): calculated for C₁₀₃H₁₆₉N₃₃O₃₃: 2397.68; measured (m/z): [M+3H]³⁺ = 799.89, [M+2H]²⁺ = 1199.22.

### (5) Synthesis of L-12

A peptide sequence was constructed from a Fmoc-NH-SAL resin (439 mg, 0.25 mmol) by the standard procedure for Fmoc-SPPS described above. After condensation of Fmoc-Arg(Pbf)-OH, Fmoc-MeDbz-OH, and Fmoc-Ala-OH in this order, the peptide chain was elongated by microwave-assisted Fmoc-SPPS. For coupling of the N-terminal cysteine, Boc-Cys(Acm)-OH was used. The resin with the peptide chain constructed was treated with 50 mM 4-nitrophenyl chloroformate in DCM (25 mL) for 1 hour and then treated with 0.5 M (i-Pr)₂NEt for 15 minutes in DMF (25 mL). It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. After the precipitate obtained was dissolved in a 0.1% TFA-containing 50% CH₃CN solution, the crude product was purified by HPLC, to obtain a desired peptide L-12 (255 mg, yield: 29%, SEQ ID NO: 66).
MS (ESI): calculated for C₁₅₃H₂₂₉N₄₉O₄₀S₂: 3458.94; measured (m/z): [M+5H]⁵⁺ = 692.18, [M+4H]⁴⁺ = 865.48, [M+3H]³⁺ = 1153.70.

### (6) Synthesis of L-13

A peptide sequence was constructed from a Fmoc-NH-SAL PEG resin (91 mg, 0.02 mmol) by the standard procedure for Fmoc-SPPS described above. After condensation of Fmoc-Arg(Pbf)-OH, Fmoc-Dbz-OH, and Fmoc-Tyr(tBu)-OH in this order, the peptide chain was elongated by Fmoc-SPPS. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. After the precipitate obtained was dissolved in a 0.1% TFA-containing 50% CH₃CN solution, the crude product was purified by HPLC, to obtain a desired peptide L-13 (3.1 mg, yield: 2.8%, SEQ ID NO: 67) .
MS (ESI) : calculated for C₂₄₁H₃₇₄N₇₂O₇₅S₃: 5576.25; measured (m/z): [M+7H]⁷⁺ = 797.20, [M+6H]⁶⁺ = 930.27, [M+5H]⁵⁺ = 1116.27, [M+4H]⁴⁺ = 1394.60, [M+3H]³⁺ = 1858.98.

### (7) Synthesis of L-14

A protective peptide was constructed from a Fmoc-NH-SAL PEG resin (91 mg, 0.02 mmol) by the standard procedure for Fmoc-SPPS described above. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. The precipitate obtained was dissolved in a minimum amount of a 0.1% TFA-containing 50% CH₃CN solution. The crude product was purified by HPLC, to obtain a desired peptide L-14 (6.1 mg, 8.7% yield: SEQ ID NO: 68).
MS (ESI) : calculated for C₁₅₀H₂₃₆N₄₆O₄₉S: 3499.87; measured (m/z): [M+3H]³⁺ = 1167.23, [M+2H]²⁺ = 1750.12.

### (8) Synthesis of L-15

Thioester L-12 (17 mg, 4.8 µmol) and peptide L-13 (30 mg, 5.3 µmol) were treated in a ligation solution (400 mM MPAA, 100 mM TCEP, 6 M guanidine, 200 mM phosphate buffer: pH 7.0; 2.4 mL) at 37°C for 2 hours. The reaction was monitored by analytical HPLC. The crude product was purified by HPLC, to obtain a desired peptide L-15 (12 mg, 30% yield: SEQ ID NO: 69).
MS (ESI): calculated for C₃₇₉H₅₈₂N₁₁₄O₁₁₂S₅: 8687.81; measured (m/z): [M+11H]¹¹⁺ = 790.42, [M+10H]¹⁰⁺ = 869.51, [M+9H]⁹⁺ = 966.20, [M+8H]⁸⁺ = 1087.03, [M+7H]⁷⁺ = 1242.50, [M+6H]⁶⁺ = 1448.39, [M+5H]⁵⁺ = 1738.15.

### (9) Synthesis of L-16

Peptide L-15 (77 mg, 8.9 µmol) was treated in a desulfurization reaction solution (20 mM VA-044, 100 mM MESNa, 250 mM TCEP, 6 M guanidine, 200 mM phosphate buffer: pH 6.5; 18 mL) at 37°C for 2 hours. The reaction was monitored by analytical HPLC. The crude product was purified by HPLC, to obtain a desired peptide L-16 (63 mg, 82% yield: SEQ ID NO: 70).
MS (ESI): calculated for C₃₇₉H₅₈₂N₁₁₄O₁₁₂S₄: 8655.75; measured (m/z): [M+11H]¹¹⁺ = 787.81, [M+10H]¹⁰⁺ = 866.47, [M+9H]⁹⁺ = 962.45, [M+8H]⁸⁺ = 1083.00, [M+7H]⁷⁺ = 1237.57, [M+6H]⁶⁺ = 1442.96, [M+5H]⁵⁺ = 1731.32.

### (10) Synthesis of L-17

Peptide L-16 (61 mg, 7.0 µmol) and PdCl₂ (12 mg, 70 µmol) were reacted in a 200 mM phosphate buffer containing 6 M guanidine (3.5 mL) at 37°C for 30 minutes. Then, a small amount of DTT was added, and the supernatant was collected after centrifugation. The reaction was monitored by analytical HPLC. The crude product was purified by HPLC, to obtain a desired peptide L-17 (29 mg, yield: 47%, SEQ ID NO: 71).
MS (ESI): calculated for C₃₇₆H₅₇₇N₁₁₃O₁₁₁S₄: 8584.67; measured (m/z): [M+11H]¹¹⁺ = 781.81, [M+10H]¹⁰⁺ = 859.64, [M+9H]⁹⁺ = 955.10, [M+8H]⁸⁺ = 1074.37, [M+7H]⁷⁺ = 1227.16, [M+6H]⁶⁺ = 1431.14, [M+5H]⁵⁺ = 1717.18.

### (11) Synthesis of L-18

Thioester L-11 (22 mg, 9.3 µmol) and peptide L-17 (27 mg, 3.1 µmol) were reacted in a ligation buffer (100 mM MPAA, 50 mM TCEP, 6 M guanidine, 200 mM phosphate buffer: pH 7.0; 1.5 mL) at 37°C for 1 hour. The reaction was monitored by analytical HPLC. The crude product was purified by HPLC, to obtain a desired peptide L-18 (21 mg, yield: 64%, SEQ ID NO: 72).
MS (ESI): calculated for C₄₆₅H₇₂₇N₁₃₉O₁₄₁S₄: 10649.00; measured (m/z): [M+13H]¹³⁺ = 819.98, [M+12H]¹²⁺ = 888.21, [M+11H]¹¹⁺ = 968.84, [M+10H]¹⁰⁺ = 1065.79, [M+9H]⁹⁺ = 1184.43, [M+8H]⁸⁺ = 1331.65, [M+7H]⁷⁺ = 1521.62, [M+6H]⁶⁺ = 1774.94.

### (12) Synthesis of L-19

Peptide L-18 (2.2 mg, 0.21 **µ**mol) was activated in an activation solution (133 mM NaNO₂, 6 M guanidine, 200 mM phosphate buffer: pH 3.0; 78 **µ**L) at -20°C for 30 minutes. A ligation solution (400 mM MPAA, 100 mM TCEP, 6 M guanidine, 200 mM phosphate buffer: pH 7.0; 26 µL) was added thereto, to thioesterify the mixture. The reaction was monitored by analytical HPLC. The crude product was purified by HPLC, to obtain a desired peptide L-19 (1.1 mg, yield: 52%, SEQ ID NO: 73).
MS (ESI): calculated for C₄₆₀H₇₁₄N₁₃₂O₁₄₁S₅: 10509.86; measured (m/z): [M+11H]¹¹⁺ = 956.78, [M+10H]¹⁰⁺ = 1051.81, [M+9H]⁹⁺ = 1168.81, [M+8H]⁸⁺ = 1314.32, [M+7H]⁷⁺ = 1502.25, [M+6H]⁶⁺ = 1751.85.

### (13) Synthesis of L-20 (PMP12A2h1)

Thioester L-19 (1.1 mg, 0.11 **µ**mol) and peptide L-14 (0.84 mg, 0.24 **µ**mol) were reacted in a ligation solution (400 mM MPAA, 100 mM TCEP, 6 M guanidine, 200 mM phosphate buffer: pH 7.0; 55 µL) at 37°C for 2 hours. The reaction was monitored by analytical HPLC. The crude product was purified by HPLC, to obtain a desired peptide L-20 (1.0 mg, yield: 68%, SEQ ID NO: 74).
MS (ESI): calculated for C₆₀₂H₉₄₂N₁₇₈O₁₈₈S₅: 13841.52; measured (m/z): [M+15H]¹⁵⁺ = 923.60, [M+14H]¹⁴⁺ = 989.55, [M+13H]¹³⁺ = 1065.64, [M+12H]¹²⁺ = 1154.64, [M+11H]¹¹⁺ = 1259.18, [M+10H]¹⁰⁺ = 1384.96, [M+9H]⁹⁺ = 1538.53, [M+8H]⁸⁺ = 1731.24.

### (14) Synthesis of D-11

D-11 was obtained (115 mg, yield: 19%, SEQ ID NO: 65 (all amino acids are D-forms except achiral glycine; the same applies to the following description)) by the same process as in the synthesis of L-11 described above.
MS (ESI): calculated for C₁₀₃H₁₆₉N₃₃O₃₃: 2397.68; measured (m/z): [M+3H]³⁺ = 799.96, [M+2H]²⁺ = 1199.68.

### (15) Synthesis of D-12

D-12 was obtained (268 mg, yield: 31%, SEQ ID NO: 66) by the same process as in the synthesis of L-12 described above.
MS (ESI): calculated for C₁₅₃H₂₂₉N₄₉O₄₀S₂: 3458.94; measured (m/z): [M+5H]⁵⁺ = 692.59, [M+4H]⁴⁺ = 865.69, [M+3H]³⁺ = 1153.56.

### (16) Synthesis of D-13

It was synthesized by a similar process to L-13 described above. A peptide sequence was constructed from a NovaSyn TGR resin (4.0 g, 1.0 mmol) by the standard procedure for Fmoc-SPPS described above. After condensation of Fmoc-D-Arg(Pbf)-OH, Fmoc-(o-Boc)Dbz-OH, and Fmoc-D-Tyr(tBu)-OH in this order, the peptide chain for 20 residues was elongated by microwave-assisted Fmoc-SPPS and then by standard Fmoc-SPPS. It was deprotected and cleaved from the resin with TFA/H₂O/m-cresol/thioanisole/EDT (80:5:5:5:5) for 2 hours. After removing the resin by filtration, the crude product was precipitated and washed with cold Et₂O. After the precipitate obtained was dissolved in a 0.1% TFA-containing 50% CH₃CN solution, the crude product was purified by HPLC, to obtain a desired peptide D-13 (307 mg, yield: 5.5%, SEQ ID NO: 67).
MS (ESI) : calculated for C₂₄₁H₃₇₄N₇₂O₇₅S₃: 5576.25; measured (m/z): [M+6H]⁶⁺ = 930.51, [M+5H]⁵⁺ = 1116.49, [M+4H]⁴⁺ = 1394.47, [M+3H]³⁺ = 1859.18.

### (17) Synthesis of D-14

D-14 was obtained (12 mg, yield: 6.1%, SEQ ID NO: 68) by the same process as in the synthesis of L-14 described above.
MS (ESI) : calculated for C₁₅₀H₂₃₆N₄₆O₄₉S: 3499.87; measured (m/z): [M+3H]³⁺ = 1167.42, [M+2H]²⁺ = 1750.46.

### (18) Synthesis of D-15

D-15 was obtained (139 mg, yield: 45%, SEQ ID NO: 69) by the same process as in the synthesis of L-15 described above.
MS (ESI): calculated for C₃₇₉H₅₈₂N₁₁₄O₁₁₂S₅: 8687.81; measured (m/z): [M+10H]¹⁰⁺ = 869.77, [M+9H]⁹⁺ = 965.86, [M+8H]⁸⁺ = 1086.95, [M+7H]⁷⁺ = 1241.88, [M+6H]⁶⁺ = 1449.03, [M+5H]⁵⁺ = 1738.06.

### (19) Synthesis of D-16

D-16 was obtained (71 mg, yield: 75%, SEQ ID NO: 70) from D-15 (95 mg, 11 µmol) by the same process as in the synthesis of L-16 described above.
MS (ESI): calculated for C₃₇₉H₅₈₂N,₁₄0₁₁₂S₄: 8655.75; measured (m/z): [M+11H]¹¹⁺ = 787.75, [M+10H]¹⁰⁺ = 866.42, [M+9H]⁹⁺ = 962.74, [M+8H]⁸⁺ = 1082.79, [M+7H]⁷⁺ = 1237.29, [M+6H]⁶⁺ = 1443.20, [M+5H]⁵⁺ = 1731.76.

### (20) Synthesis of D-17

D-17 was obtained (39 mg, yield: 76%, SEQ ID NO: 71) by the same process as in the synthesis of L-17 described above.
MS (ESI): calculated for C₃₇₆H₅₇₇N₁₁₃O₁₁₁S₄: 8584.67; measured (m/z): [M+11H]¹¹⁺ = 781.13, [M+10H]¹⁰⁺ = 859.26, [M+9H]⁹⁺ = 954.63, [M+8H]⁸⁺ = 1074.18, [M+7H]⁷⁺ = 1227.17, [M+6H]⁶⁺ = 1431.62, [M+5H]⁵⁺ = 1717.09.

### (21) Synthesis of D-18

D-18 was obtained (39 mg, yield: 70%, SEQ ID NO: 72) by the same process as in the synthesis of L-18 described above.
MS (ESI): calculated for C₄₆₅H₇₂₇N₁₃₉O₁₄₁S₄: 10649.00; measured (m/z): [M+13H]¹³⁺ = 819.55, [M+12H]¹²⁺ = 888.24, [M+11H]¹¹⁺ = 968.96, [M+10H]¹⁰⁺ = 1065.32, [M+9H]⁹⁺ = 1184.22, [M+8H]⁸⁺ = 1331.80, [M+7H]⁷⁺ = 1522.39, [M+6H]⁶⁺ = 1775.59.

### (22) Synthesis of D-19

D-19 was obtained (10 mg, yield: 58%, SEQ ID NO: 73) by the same process as in the synthesis of L-19 described above.
MS (ESI): calculated for C₄₆₀H₇₁₄N₁₃₂O₁₄₁S₅: 10509.86; measured (m/z): [M+11H]¹¹⁺ = 955.88, [M+10H]¹⁰⁺ = 1051.94, [M+9H]⁹⁺ = 1168.23, [M+8H]⁸⁺ = 1314.67, [M+7H]⁷⁺ = 1502.28, [M+6H]⁶⁺ = 1751.97.

### (23) Synthesis of D-20 (D-PMP12A2h1)

D-20 was obtained (2.6 mg, yield: 40%, SEQ ID NO: 74) by the same process as in the synthesis of L-20 described above.
MS (ESI): calculated for C₆₀₂H₉₄₂N₁₇₈O₁₈₈S₅: 13841.52; measured (m/z): [M+15H]¹⁵⁺ = 923.72, [M+14H]¹⁴⁺ = 989.70, [M+13H]¹³⁺ = 1065.83, [M+12H]¹²⁺ = 1154.30, [M+11H]¹¹⁺ = 1259.49, [M+10H]¹⁰⁺ = 1384.91, [M+9H]⁹⁺ = 1539.04, [M+8H]⁸⁺ = 1731.03.

### (24) Folding of PMP12A2h1 and D-PMP12A2h1

Peptide L-20 or D-20 was dissolved in PBS (pH 7.4) containing 6 M guanidine and 40 mM DTT to 1.0 mg/mL and incubated at room temperature for 2 hours. This solution was diluted 100-fold with PBS (pH 7.4) and allowed to stand overnight at room temperature. The solution was concentrated using MWCQ3000 centrifugal filtration membrane (Millipore, Amicon-Ultra3kDa). Subsequently, 10 volumes of 5,5'-dithiobis(2-benzoic acid) (DTNB) were added and incubated at 37°C for 5 hours. A MWCO3000 centrifugal filtration membrane was used to purify the target protein.

### (25) CD spectra of PMP12A2h1 and D-PMP12A2h1

Folded pmp12a2h1 and D-PMP12A2h1 were diluted with PBS (pH 7.4) and adjusted to a concentration of 10 µM each. CD spectra of each protein were recorded at 20°C using a circular dichroism spectrometer (JACSO J-720).

The CD spectrum of pmp12a2h1 has a minimum at 216 nm and a maximum at 203 nm, indicating the presence of a β-sheet-like structure (Figure 10). The CD spectrum of D-PMP12A2h1 is a sign-opposite of the CD spectrum of pmp12a2h1, suggesting that D-PMP12A2h1 is folded into a conformation antipodal to PMP12A2h1.

### (26) Surface plasmon resonance (SPR) analysis of PMP12A2h1

Experiments were performed using a Biacore X100 (Cytiva). A recombinant vWF A1 domain (U-Protein Express BV, V0002) was immobilized on a CM5 chip at 2500 RU. Folded PMP12A2h1 was diluted with HBS-EP buffer and added. The running buffer was HBS-EP buffer, the dissociation solution was 1 M NaCl, the analyte contact time was 3 minutes, the dissociation time was 7 minutes, and the flow rate was 30 µL/min. Triplicate assays were performed, and the dissociation constant was calculated from steady-state binding.

Chemically synthesized pmp12a2h1 was shown to bind to the vWF A1 domain with sufficient binding affinity (Figure 11). It suggests that the synthetic pmp12a2h1 has appropriate biological activity.

### Example 3

A T7 phage library with randomized CDR regions is used to screen for the VHH antibody composed of D-amino acids specific to the target molecule.

### (1) Phage library

Of the VHH antibody (PMP12A2h1) sequences of Caplacizumab, T7 phage library in which only CDR3 or all three regions of CDR1, CDR2, and CDR3 are randomized is used. At this time, libraries with CDR3 random amino acid lengths of 7, 10, 13, 17, and 20-mers are used.

### (2) Target molecule

As a target molecule, a molecule that can be chemically synthesized (for example, cytokine and chemokine) is selected by a reported protocol, and a mirror-image protein synthesized using D-amino acids is used. At this time, a tag such as 6 × His is added to the N-terminus or C-terminus of the mirror-image protein.

### (3) Phage screening

The target mirror-image protein is dissolved in tris buffer TBS (50 mM Tris-HCl pH 7.5, 150 mM NaCl) to a concentration of 1 to 10 µg/ml, and a Nickel plate (Thermo Scientific) is coated therewith at room temperature for 30 minutes (100 µl/well). Thereafter, blocking is performed using a blocking agent (such as 3% BSA and 4% BlockAce) at room temperature for 2 hours. After washing the wells with PBST three times, 100 µl of the phage library described above (10⁹ to 10¹⁰ pfu) is added and incubated at room temperature for 30 minutes. The wells are washed with a washing solution (PBST) 10 times, to remove unbound phages. Thereafter, 100 µl of elution buffer (500 mM imidazole-containing TBS) is added and incubated for about 2 minutes, and the target molecule and phages bound specifically thereto are eluted. The eluate is added to 10 ml LB medium (available from NACALAI TESQUE, INC.) containing E. coli (BLT5403), followed by shaking at 37 °C for 1.5 to 2 hours (190 r/min) until E. coli is lysed. The phages added to the wells and the recovered phages are partially plated on an accept petri dish (As One) together with 4 ml top agar (0.75% LB agar medium, NACALAI TESQUE, INC.), and the number of plaques formed is counted. The binding efficiency (concentration factor) is calculated from this value. The above steps are repeated 3 to 4 times to see if the binding efficiency increases with each round.

### (4) Sequence confirmation

Clones are obtained from the lysed phage fraction in which enrichment is observed, and the random sequences of VHHs are individually obtained by Sanger sequencing analysis (ABI 3130 Genetic analyzer). In addition, a next-generation sequencer (Illumina iSeq 100 system) is used to comprehensively obtain the sequence of the random region. VHH antibodies with sequences showing high homology in random regions are targeted for synthesis.

### (5) Synthesis of target-specific D-VHH antibodies

D-VHH antibodies with CDR sequences identified of interest are synthesized according to the procedures of the invention. Affinity with the target molecule consisting of the original L-amino acids is analyzed using the Biacore system.

### Industrial Applicability

The low-molecular antibody of the present invention has reduced immunogenicity while having specificity similar to that of natural antibody molecules. Therefore, it is useful in the medical field such as drugs or diagnosis/imaging.

All prior art documents cited herein are hereby incorporated by reference.

### Sequence Listing Free Text

SEQ ID NO: 55 - Synthetic peptide L-1/D-1
SEQ ID NO: 56 - Synthetic peptide L-2/D-2
SEQ ID NO: 57 - Synthetic peptide L-3/D-3
SEQ ID NO: 58 - Synthetic peptide L-4/D-4
SEQ ID NO: 59 - Synthetic peptide L-5/D-5
SEQ ID NO: 60 - Synthetic peptide L-6/D-6
SEQ ID NO: 61 - Synthetic peptide L-7/D-7
SEQ ID NO: 62 - Synthetic peptide L-8/D-8
SEQ ID NO: 63 - Synthetic peptide L-9/D-9
SEQ ID NO: 64 - Synthetic peptide L-10/D-10
SEQ ID NO: 65 - Synthetic peptide L-11/D-11
SEQ ID NO: 66 - Synthetic peptide L-12/D-12
SEQ ID NO: 67 - Synthetic peptide L-13/D-13
SEQ ID NO: 68 - Synthetic peptide L-14/D-14
SEQ ID NO: 69 - Synthetic peptide L-15/D-15
SEQ ID NO: 70 - Synthetic peptide L-16/D-16
SEQ ID NO: 71 - Synthetic peptide L-17/D-17
SEQ ID NO: 72 - Synthetic peptide L-18/D-18
SEQ ID NO: 73 - Synthetic peptide L-19/D-19
SEQ ID NO: 74 - Synthetic peptide L-20/D-20

## Claims

1. A low-molecular antibody composed of D-amino acids and achiral glycine, wherein the low-molecular antibody has a molecular weight of 60 KDa or less.

2. The low-molecular antibody according to claim 1, wherein the low-molecular antibody is any one selected from a single-domain antibody, scFV, and Fab.

3. The low-molecular antibody according to claim 1, wherein the low-molecular antibody is a VHH antibody.

4. The low-molecular antibody according to claim 1, wherein the low-molecular antibody is a VHH antibody having:
(1) four framework regions represented by the amino acid sequences set forth in SEQ ID NOs: 1 to 4 or amino acid sequences having an identity of at least 60% with the aforementioned sequences;
(2) four framework regions represented by the amino acid sequences set forth in SEQ ID NOs: 5 to 8 or amino acid sequences having an identity of at least 60% with the aforementioned sequences;
(3) four framework regions represented by the amino acid sequences set forth in SEQ ID NOs: 9 to 12 or amino acid sequences having an identity of at least 60% with the aforementioned sequences;
(4) four framework regions represented by the amino acid sequences set forth in SEQ ID NOs: 13 to 16 or amino acid sequences having an identity of at least 60% with the aforementioned sequences; or
(5) four framework regions represented by the amino acid sequences set forth in SEQ ID NOs: 17 to 20 or amino acid sequences having an identity of at least 60% with the aforementioned sequences.

5. A method for producing a low-molecular antibody composed of D-amino acids and achiral glycine, the method comprising:
1) dividing an amino acid sequence of a low-molecular antibody into a plurality of segments having a cysteine residue or an alanine residue at the N-terminus;
2) chemically synthesizing each of the segments by the solid-phase method; and
3) sequentially ligating the synthesized segments by the NCL method to synthesize a low-molecular antibody composed of D-amino acids and achiral glycine.

6. The method according to claim 5, wherein the low-molecular antibody is any one selected from a single-domain antibody, scFV, and Fab.

7. The method according to claim 5, wherein the low-molecular antibody is a VHH antibody.

8. A method for producing a low-molecular antibody composed of D-amino acids and achiral glycine against a target protein, the method comprising:
1) providing a mirror-image target protein composed of D-amino acids and achiral glycine against a target protein;
2) screening an antibody library using the mirror-image target protein, to obtain an antibody having affinity to the mirror-image target protein; and
3) synthesizing a low-molecular antibody composed of D-amino acids and achiral glycine based on an amino acid sequence of the obtained antibody.

9. The method according to claim 8, wherein synthesis of low-molecular antibody composed of D-amino acids and achiral glycine comprises the following steps:
1) dividing an amino acid sequence of a low-molecular antibody into a plurality of segments having a cysteine residue or an alanine residue at the N-terminus;
2) chemically synthesizing each of the segments by the solid-phase method; and
3) sequentially ligating the synthesized segments by the NCL method to synthesize a low-molecular antibody composed of D-amino acids and achiral glycine.

10. The method according to claim 8, wherein the low-molecular antibody is any one selected from a single-domain antibody, scFV, and Fab.

11. The method according to claim 8, wherein the low-molecular antibody is a VHH antibody.

12. A polyvalent or a multi-specific antibody obtained by linking the low-molecular antibodies according to any one of claims 1 to 4.

13. A method for producing a polyvalent or a multi-specific antibody composed of D-amino acids, comprising linking low-molecular antibodies produced by the method according to any one of claims 5 to 11.

14. A kit for producing a polyvalent or a multi-specific antibody composed of D-amino acids, the kit comprising one or a plurality of polypeptides each having the amino acid sequence (a) or (b) below and composed of D-amino acids and achiral glycine:
(a) an amino acid sequence set forth in any one of SEQ ID NOs: 55 to 57, 61, and 63; or
(b) an amino acid sequence having an identity of at least 60% with the amino acid sequence set forth in any one of SEQ ID NOs: 55 to 57, 61, and 63.
